# NEW EUROPEAN PATENT SPECIFICATION

(11) **EP 0 122 791 B2**
(45) Date of publication and mention of the opposition decision: **02.04.1997**
(45) Mention of the grant of the patent: 29.03.1989
(21) Application number: 84302533.9
(22) Date of filing: 13.04.1984
(51) Int. Cl.: C12N 15/00, C12N 5/00

(54) **Plant gene expression**
Pflanzengen-Expression
Expression de gène végétal

(30) Priority: 15.04.1983 US 485613
(43) Date of publication of application: 24.10.1984
(73) Proprietor: Mycogen Plant Science, Inc., Wilmington Delaware 19801 (US)
(72) Inventor: Hall, Timothy C., Madison, WI 53711 (US); Kemp, John D., Madison, WI 53711 (US); Slightom, Jerry L., Madison, WI 53714 (US); Sutton, Dennis W., McFarland, WI 53558 (US); MURAI, Norimoto, Madison Wisconsin 53703 (US)
(74) Representative: De Minvielle-Devaux, Ian Benedict Peter

(56) References cited:
- EP-A- 0 045 573
- EP-A- 0 099 255
- MOLECULAR & GENERAL GENETICS, vol. 163, 1978, Heidelberg; M. HOLSTERS et al: "Transfection and Transformation of Agrobacterium tumefaciens", pp. 181-187
- NATURE, vol. 196, no. 5852, MARCH 1982,New York; F.A. KRENS et al: "In vitro transformation of plant protoplasts with Ti-plasmid DNA", pp. 72-74
- CELL, vol. 27, no. 1, November 1981 (Part 2), Cambridge, Mass.; D.J. GARFINKEL et al: "Genetic analysis of crown gall: "Fine structure map of the T-DNA by site-directed mutagenesis", pp. 143-153

## Description

### Background

Shuttle vectors, developed by Ruvkun and Ausubel (1981) Nature *289*:85-88, provide a way to insert foreign genetic materials into positions of choice in a large plasmid, virus, or genome. There are two main problems encountered when dealing with large plasmids or genomes. Firstly, the large plasmids may have many sites for each restriction enzyme. Unique, site-specific cleavage reactions are not reproducible and multi-site cleavage reactions followed by ligation lead to great difficulties due to the scrambling of the many fragments whose order and orientation one does not want changed. Secondly, the transformation efficiency with large DNA plasmids is very low. Shuttle vectors allow one to overcome these difficulties by facilitating the insertion, often *in vitro,* of the foreign genetic material into a smaller plasmid, then transferring, usually by *in vivo* techniques, to the larger plasmid.

A shuttle vector consists of a DNA molecule, usually a plasmid, capable of being introduced into the ultimate recipient bacteria. It also includes a copy of the fragment of the recipient genome into which the foreign genetic material is to be inserted and a DNA segment coding for a selectable trait, which is also inserted into the recipient genome fragment. The selectable trait ("marker") is conveniently inserted by transposon mutagenesis or by restriction enzymes and ligases.

The shuttle vector can be introduced into the ultimate recipient cell typically a bacterium of the genus *Agrobacterium* by a tri-parental mating (Ruvkun and Ausubel, *supra),* direct transfer of a self-mobilizable vector in a bi-parental mating, direct uptake of exogenous DNA *by Agrobacterium* cells ("transformation", using the conditions of M. Holsters *et al.* (1978) Molec. Gen. Genet. *163*:181-187), by spheroplast fusion of *Agrobacterium* with another bacterial cell, by uptake of liposome-encapsulated DNA, or infection with a shuttle vector that is based on a virus that is capable of being packaged *in vitro.* A tri-parental mating involves the mating of a strain containing a mobilizable plasmid, which carries genes for plasmid mobilization and conjugative transfer, with the strain containing the shuttle vector. If the shuttle vector is capable of being mobilized by the plasmid genes, the shuttle vector is transferred to the recipient cell containing the large genome, e.g. the Ti or Ri plasmids of *Agrobacterium* strains.

After the shuttle vector is introduced into the recipient cell, possible events include a double cross over with one recombinational event on either side of the marker. This event will result in transfer of a DNA segment containing the marker to the recipient genome replacing a homologous segment lacking the insert. To select for cells that have lost the original shuttle vector, the shuttle vector must be incapable of replicating in the ultimate host cell or be incompatible with an independently selectable plasmid pre-existing in the recipient cell. One common means of arranging this is to provide in the third parent another plasmid which is incompatible with the shuttle vector and which carries a different drug resistance marker. Therefore, when one selects for resistance to both drugs, the only surviving cells are those in which the marker on the shuttle vector has recombined with the recipient genome. If the shuttle vector carries an extra marker, one can then screen for and discard cells that are the result of a single cross-over between the shuttle vector and the recipient plasmid resulting in cointegrates in which the entire shuttle vector is integrated with the recipient plasmid. If the foreign genetic material is inserted into or adjacent to the marker that is selected for, it will also be integrated into the recipient plasmid as a result of the same double recombination. It might also be carried along when inserted into the homologous fragment at a spot not within or adjacent to the marker, but the greater the distance separating the foreign genetic material from the marker, the more likely will be a recombinational event occurring between the foreign genetic material and marker, preventing transfer of the foreign genetic material.

Shuttle vectors have proved useful in manipulation of *Agrobacterium* plasmids: see D. J. Garfinkel *et al*. (1981) Cell *27*:143-153, A. J. M. Matzke and M. D. Chilton (1981) J. Molec. Appl. Genet. *1*:39-49, and J. Leemans *et al.* (1981) J. Molec. Appl. Genet. *1*:149-164, who referred to shuttle vectors by the term "intermediate vectors".

### Agrobacterium-Overview

Included within the gram-negative bacterial family Rhizobiaceae in the genus *Agrobacterium* are the species *A. tumefaciens* and *A. rhizogenes.* These species are respectively the causal agents of crown gall disease and hairy root disease of plants. Crown gall is characterized by the growth of a gall of dedifferentiated tissue. Hairy root is a teratoma characterized by inappropriate induction of roots in infected tissue. In both diseases, the inappropriately growing plant tissue usually produces one or more amino acid derivatives, known as opines, not normally produced by the plant which are catabolized by the infecting bacteria. Known opines have been classified into three families whose type members are octopine, nopaline, and agropine. The cells of inappropriately growing tissues can be grown in culture, and, under appropriate conditions, be regenerated into whole plants that retain certain transformed phenotypes.

Virulent strains of *Agrobacterium* harbor large plasmids known as Ti (tumor-inducing) plasmids in *A*. *tumefaciens* and Ri (root-inducing) plasmids in *A. rhizogenes.* Curing a strain of these plasmids results in a loss of pathogenicity. The Ti plasmid contains a region, referred to as T-DNA (transferred-DNA), which in tumors is found to be integrated into the genome of the host plant. The T-DNA encodes several transcripts. Mutational studies have shown that some of these are involved in induction of tumorous growth. Mutants in the genes for *tml, tmr,* and *tms,* respectively result in large tumors (in tobacco), a propensity to generate roots, and a tendency for shoot induction. The T-DNA also encodes the gene for at least one opine synthetase, and the Ti plasmids are often classified by the opine which they caused to be synthesized. Each of the T-DNA genes is under control of a T-DNA promoter. The T-DNA promoters resemble eukaryotic promoters in structure, and they appear to function only in the transformed plant cell. The Ti plasmid also carries genes outside the T-DNA region. These genes are involved in functions which include opine catabolism, oncogenicity, agrocin sensitivity, replication, and autotransfer to bacterial cells. The Ri plasmid is organized in a fashion analogous to the Ti plasmid. The set of genes and DNA sequences responsible for transforming the plant cell are hereinafter collectively referred to as the transformation-inducing principle (TIP). The designation TIP therefore includes both Ti and Ri plasmids. The integrated segment of a TIP is termed herein "T-DNA", whether derived from a Ti plasmid or an Ri plasmid. Recent general reviews of *Agrobacterium-caused* disease include those by D. J. Merlo (1982), Adv. Plant Pathol. *1*:139-178 L. W. Ream and M. P. Gordon (1982), Science *218*:854-859, and M. W. Bevan and M. D. Chilton (1982), Ann. Rev. Genet. *16*:357-384; G. Kahl and J. Schell (1982) *Molecular Biology of Plant Tumors.*

### Agrobacterium-Infection of plant tissues

Plant cells can be transformed by *Agrobacterium* in a number of methods known in the art which include but are not limited to co-cultivation of plant cells in culture with *Agrobacterium,* direct infection of a plant, fusion of plant protoplasts with *Agrobacterium* spheroplasts, direct transformation by uptake of free DNA by plant cell protoplasts, transformation of protoplasts having partly regenerated cell walls with intact bacteria, transformation of protoplasts by liposomes containing T-DNA, use of a virus to carry in the T-DNA, microinjection, and the like. Any method will suffice as long as the gene is reliably expressed, and is stably transmitted through mitosis and meiosis.

The infection of plant tissue by *Agrobacterium* is a simple technique well known to those skilled in the art (for an example, see D. N. Butcher *et al.* (1980) in *Tissue Culture Methods for Plant Pathologists,* eds.: D. S. Ingrams and J. P. Helgeson, pp. 203-208). Typically a plant is wounded by any of a number of ways, which include cutting with a razor, puncturing with a needle, or rubbing with abrasive. The wound is then inoculated with a solution containing tumor-inducing bacteria. An alternative to the infection of intact plants is the inoculation of pieces of tissues such as potato tuber disks (D. K. Anand and G. T. Heberlein (1977) Amer. J. Bot. *64*:153-158) or segments of tobacco stems (Binns, *et al.*). After induction, the tumors can be placed in tissue culture on media lacking phytohormones. Hormone independent growth is typical of transformed plant tissue and is in great contrast to the usual conditions of growth of such tissue in culture (A. C. Braun (1956) Cancer Res. *16*:53-56).

*Agrobacterium* is also capable of infecting isolated cells and cells grown in culture, Marton *et al.* (1979) Nature *277*:129-131, and isolated tobacco mesophyll protoplasts. In the latter technique, after allowing time for partial regeneration of new cell walls, *Agrobacterium* cells were added to the culture for a time and then killed by the addition of antibiotics. Only those cells exposed to *A. tumefaciens* cells harboring the Ti plasmid were capable of forming calli when plated on media lacking hormone. Most calli were found to contain an enzymatic activity involved in opine anabolism. Other workers (R. B. Horsch and R. T. Fraley (18 January 1983) 15th Miami Winter Symposium) have reported transformations by co-cultivation, leading to a high rate (greater than 10%) of calli displaying hormone-independent growth, with 95% of those calli making opines, M. R. Davey *et al.* (1980) in Ingram and Helgeson, *supra,* pp. 209-219, describe the infection of older cells that had been regenerated from protoplasts.

Plant protoplasts can be transformed by the direct uptake of TIP plasmids. M. R. Davey *et al.* (1980) Plant Sci. Lett. *18*:307-313, and M. R. Davey *et al.* (1980) in Ingram and Helgeson, *supra,* were able to transform *Petunia* protoplasts with the Ti plasmid in the presence of poly-L-and ornithine to a phenotype of opine synthesis and hormone-independent growth in culture. It was later shown (J. Draper *et al.* (1982) Plant and Cell Physiol. *23*:451-458, M. R. Davey *et al.* (1982) in *Plant Tissue Culture 1982,* ed: A. Fujiwara, pp. 515-516) that polyethylene glycol stimulated Ti uptake and that some T-DNA sequences were integrated into the genome. F. A. Krens *et al.* (1982) Nature *296*:72-74, reported similar results using polyethylene glycol following by a calcium shock, though their data suggests that the integrated T-DNA included flanking Ti plasmid sequences.

An alternative method to obtain DNA uptake involves the use of liposomes. The preparation of DNA containing liposomes is taught by Papahadjopoulos in US Patents 4,078,052 and 4,235,871. Preparations for the introduction of Ti-DNA *via* liposomes have been reported (T. Nagata *et al.* (1982) in Fujiwara, *supra,* pp. 509-510, and T. Nagata (1981) Mol. Gen. Genet. *184*:161-165). An analogous system involves the fusion of plant and bacterial cells after removal of their cell walls. An example of this technique is the transformation of *Vinca* protoplast *by Agrobacterium* spheroplasts reported by S. Hasezawa *et al.* (1981) Mol. Gen. Genet. *182*:206-210. Plant protoplasts can take up cell wall delimited *Agrobacterium* cells (S. Hasezawa *et al.* (1982) in Fujiwara, *supra* pp. 517-518).

T-DNA can be transmitted to tissue regenerated from a fusion of two protoplasts, only one of which had been transformed (G. J. Wullems *et al.* (1980) Theor. Appl. Genet. *56*:203-208). As detailed in the section on Regeneration of Plants, T-DNA can pass through meiosis and be transmitted to progeny as a simple Mendelian trait.

### Agrobacterium-Regeneration of plants

Differentiated plant tissues with normal morphology have been obtained from crown gall tumors. A. C. Braun and H. N. Wood (1976) Proc. Natl. Acad. Sci. USA *73*:493-500, grafted tobacco teratomas onto normal plants and were able to obtain normally appearing shoots which could flower. The shoots retained the ability to make opines and to grow independently of phytohormones when placed in culture. In the plants screened, these tumor phenotypes were not observed to be transmitted to progeny, apparently being lost during meiosis (R. Turgeon *et al.* (1976) Proc. Natl. Acad. Sci. USA *73*:3562-3564). Plants which had spontaneously lost tumorous properties, or which were derived from teratoma seed, were initially shown to have lost all their T-DNA (F.-M. Yang *et al.* (1980) In Vitro *16*:87-92, F. Yang *et al.* (1980) Molec. Gen. Genet. *177*:707-714, M. Lemmers *et al.* (1980) J. Mol. Biol. *144*:353-376). However, later work with plants that had become revertants after hormone treatment (1 mg/l kinetin) showed that plants which had gone through meiosis, though losing T-DNA genes responsible for the transformed phenotype, could retain sequences homologous to both ends of T-DNA (F. Yang and R. B. Simpson (1981) Proc. Natl. Acad. Sci. USA *78*:4151-4155). G. J. Wullems *et al.* (1981) Cell *24*:719-724, further demonstrated that genes involved in opine anabolism were capable of passing through meiosis though the plants were male sterile and that seemingly unaltered T-DNA could be inherited in a Mendelian fashion (G. Wullems *et al.* (1982) in A. Fujiwara, *supra).* L. Otten *et al.* (1981) Molec. Gen. Genet. *183*:209-213, used Tn7 transposon-generated Ti plasmid mutants in the *tms* (shoot-inducing) locus to create tumors which proliferated shoots. When these shoots were regenerated into plants, they were found to form self-fertile flowers. The resultant seeds germinated into plants which contained T-DNA and made opines. Similar experiments with a *tmr* (root-inducing) mutant showed that full-length T-DNA could be transmitted through meiosis to progeny, that in those progeny nopaline genes could be expressed, though at variable levels, and that cotransformed yeast alcohol dehydrogenase I gene was not expressed (K. A. Barton *et al.* (1983) Cell *32*:1033-1043). It now appears that regenerated tissues which lack T-DNA sequences are probably descended from untransformed cells which "contaminate" the tumor (G. Ooms et *al.* (1982) Cell *30*:589-597).

Roots resulting from transformation from *A. rhizogenes* have proven relatively easy to regenerate into plantlets (M.-D. Chilton *et al*. (1982) Nature *295*:432-434.

### Agrobacterium-Genes on the TIP plasmids

A number of genes have been identified within the T-DNA of the TIP plasmids. About half a dozen octopine plasmid T-DNA transcripts have been mapped (S. B. Gelvin *et al.* (1982) Proc. Natl. Acad. Sci. USA *79*:76-80, L. Willmitzer *et al*. (1982)⁻EMBO J. *1*:139-146) and some functions have been assigned (J. Leemans *et al.* (1982) EMBO J. *1*:147-152). The four genes of an octopine type plasmid that have been well defined by transposon mutagenesis include *tms*, *tmr* and *tml* (D. J. Garfinkel *et al*. (1981) Cell *27*:143-153). Ti plasmids which carry mutations in these genes respectively incite tumorous calli of *Nicotiana tabacum* which generate shoots, proliferate roots, and are larger than normal. In other hosts, mutants of these genes can induce different phenotypes (see Chilton, M. D., Ann. Rev. Genet. (1982). The phenotypes of *tms* and *tmr* are correlated with differences in the phytohormone levels present in the tumor. The differences in cytokinin:auxin ratios are similar to those which in culture induce shoot or root formation in untransformed callus tissue (D. E. Akiyoshi *et al.* (1983) Proc. Natl. Acad. Sci. USA *80*:407-411). T-DNA containing a functional gene for either *tms* or *tmr* alone, but not functional *tml* alone, can promote significant tumor growth. Promotion of shoots and roots is respectively stimulated and inhibited by functional *tml* (L. W. Ream *et al.* (1983) Proc. Natl. Acad. Sci. USA *80*:1660-1664). Mutations in T-DNA genes do not seem to affect the insertion of T-DNA into the plant genome (J. Leemans *et al.* (1982) *supra,* L. W. Ream *et al. (1983) supra).* The *ocs* gene encodes octopine synthetase, which has been sequenced by H. De Greve *et al.* (1982) J. Mol. Appl. Genet. *1*:499-511. It does not contain introns (intervening sequence commonly found in eukaryotic genes which are posttranscriptionally spliced out of the messenger precursor during maturation of the mRNA). It does have sequences that resemble a eukaryotic transcriptional signal ("TATA box") and a polyadenylation site. As plant cells containing the enzyme octopine synthetase detoxify homo-arginine, the *ocs* gene may prove to be a useful selectable marker for plant cells that have been transformed by foreign DNA (G. M. S. Van Slogteren *et al* (1982) Plant Mol. Biol. *1*:133-142).

Nopaline Ti plasmids encode the nopaline synthetase gene *(nos),* which has been sequenced by A. Depicker *et al.* (1982) J. Mol. Appl. Genet. *1*:561-573. As was found with the *ocs* gene, *nos* is not interrupted by introns. It has two putative polyadenylation sites and a potential "TATA box". In contrast to *ocs, nos* is preceeded by a sequence which may be a transcriptional signal known as a "CAT box". J. C. McPhersson *et al*. (1980) Proc. Natl. Acad. Sci. USA *77*:2666-2670, reported the *in vitro* translation of T-DNA encoded mRNAs from crown gall tissues.

Transcription from hairy root T-DNA has also been detected (L. Willmitzer *et al.* (1982) Mol. Gen. Genet. *186*:16-22). Functionally, the hairy root syndrome appears to be equivalent of a crown gall tumor incited by a Ti plasmid mutated in *tmr* (F. F. White and E. W. Nester (1980) J. Bacteriol. *144*:710-720.

In eukaryotes, methylation (especially of cytosine residues) of DNA is correlated with transcriptional inactivation; genes that are relatively undermethylated are transcribed into mRNA. Gelvin *et al.* (1983) Nucleic Acids Res. *1*:159-174, has found that the T-DNA in crown gall tumors is always present in at least one unmethylated copy. That the same genome may contain numerous other copies of T-DNA which are methylated suggests that the copies of T-DNA in excess of one may be biologically inert. (See also G. Ooms *et al.* (1982) Cell *30*:589-597).

The Ti plasmid encodes other genes which are outside of the T-DNA region and are necessary for the infection process. (See M. Holsters *et al.* (1980) Plasmid *3*:212-230 for nopaline plasmids, and H. De Greve *et al.* (1981) Plasmid *6*:235-248, D. J. Garfinkel and E. W. Nester (1980) J. Bacteriol *144*:732-743, and G. Ooms (1980) J. Bacteriol *144*:82-91 for octopine plasmids). Most important are the *onc* genes, which when mutated result in Ti plasmids incapable of oncogenecity. (These loci are also known as *vir,* for virulence.) The *onc* genes function in *trans,* being capable of causing the transformation of plant cells with T-DNA of a different plasmid type and physically located on another plasmid (J. Hille *et al.* (1982) Plasmid *7*: 107-118, H. J. Klee *et al.* (1982) J. Bacteriol *150*:327-331, M.-D. Chilton (18 January 1983) 15th Miami Winter Symp. Nopaline Ti DNA has direct repeats of about 25 base pairs immediately adjacent to the left and right borders of the T-DNA which might be involved in either excision from the Ti plasmid or integration into the host genome (N. S. Yadav *et al.* (1982) Proc. Natl. Acad. Sci. USA *79*:6322-6326), and a homologous sequence has been observed adjacent to an octopine T-DNA border (R. B. Simpson *et al.* (1982) Cell *29*:1005-1014). Opine catabolism is specified by the *ocs* and *nos* genes, respectively of octopine- and nopaline-type plasmids. The Ti plasmid also encodes functions necessary for its own reproduction including an origin of replication. Ti plasmid transcripts have been detected in *A. tumefaciens* cells by S. B. Gelvin *et al.* (1981) Plasmid *6*:17-29, who found that T-DNA regions were weakly transcribed along with non-T-DNA sequences. Ti plasmid-determined characteristics have been reviewed by Merlo, *supra* (see especially Table II), and Ream and Gordon *supra.*

### Agrobacterium-TIP plasmid DNA

Different octopine-type Ti plasmids are nearly 100% homologous to each other when examined by DNA hybridization (T. C. Currier and E. W. Nester (1976) J. Bacteriol. *126*:157-165) or restriction enzyme analysis (D. Sciaky *et al.* (1978) Plasmid *1*:238-253). Nopaline-type Ti plasmids have as little as 67% homology to each other (Currier and Nester, *supra).* A survey revealed that different Ri plasmids are very homologous to each other (P. Costantino *et al.* (1981) Plasmid *5*:170-182). N. H. Drummond and M.-D. Chilton (1978) J. Bacteriol. *136*:1178-1183, showed that proportionally small sections of octopine and nopaline type Ti plasmids were homologous to each other. These homologies were mapped in detail by G. Engler *et al.* (1981) J. Mol. Biol. *152*:183-208. They found that three of the four homologous regions were subdivided into three (overlapping the T-DNA), four (containing some *onc* genes), and nine (having *onc* genes) homologous sequences. The uninterrupted homology contains at least one *tra* gene (for conjugal transfer of the Ti plasmid to other bacterial cells), and genes involved in replication and incompatibility. This uninterrupted region has homology with a *Sym* plasmid (involved in symbiotic nitrogen fixation) from a species of *Rhizobium,* a different genus in the family Rhizobiaceae (R. K. Prakash *et al.* (1982) Plasmid *7*:271-280). The order of the four regions is not conserved, though they are all oriented in the same direction. Part of the T-DNA sequence is very highly conserved between nopaline and octopine plasmids (M.-D. Chilton *et al.* (1978) Nature *275*:147-149, A. Depicker *et al.* (1978) Nature *275*:150-153). Ri plasmids have been shown to have extensive homology among themselves, and to both octopine (F. F. White and E. W. Nester (1980) J. Bacteriol. *144*:710-720) and nopaline (G. Risuleo *et al.* (1982) Plasmid *7*:45-51) Ti plasmids, primarily in regions encoding *onc* genes. Ri T-DNA contains extensive though weak homologies to T-DNA from both types of Ti plasmid (L. Willmitzer *et al.* (1982) Mol. Gen. Genet. *186*:3193-3197). Plant DNA from uninfected *Nicotiana glauca* contains sequences, referred to as cT-DNA (cellular T-DNA), that show homology to a portion of the Ri T-DNA (F. F. White *et al.* (1983) Nature *301*:348-350).

It has been shown that a portion of the Ti (M.-D. Chilton *et al.* (1977) Cell *11*:263-271) or Ri (M.-D. Chilton (1982) Nature *295*:432-434, F. F. White *et al.* (1982) Proc. Natl. Acad. Sci. USA *79*:3193-3197, L. Willmitzer (1982) Mol. Gen. Genet. *186*:13-22) plasmid is found in the DNA of tumorous plant cells. The transferred DNA is known as T-DNA. T-DNA is integrated into the host DNA (M. F. Thomashow *et al.* (1980) Proc. Natl. Acad. Sci. USA *77*:6448-6452, N.S. Yadav *et al.* (1980) Nature *287*:458-461) in the nucleus (M. P. Nuti *et al.* (1980) Plant Sci. Lett. *18*:1-6, L. Willmitzer *et al. (1980)* Nature *287*:359-361, M.-D. Chilton *et al.* (1980) Proc. Natl. Acad. Sci. USA *77*:4060-4064).

M. F. Thomashow *et al.* (1980) Proc. Natl. Acad. Sci. USA *77*:6448-6452, and M. F. Thomashow *et al.* (1980) Cell *19*:729-739, found the T-DNA from octopine-type Ti plasmids to have been integrated in two separate sections, TL-DNA and TR-DNA, left and right T-DNAs respectively. The copy numbers of TR and TL can vary (D. J. Merlo *et al.* (1980) Molec. Gen. Genet. *177*:637-643). A core of T-DNA is highly homologous to nopaline T-DNA (Chilton *et al.* (1978) *supra* and Depicker *et al.* (1978) *supra),* is required for tumor maintenance, is found in TL, is generally present in one copy per cell, and codes for the genes *tms, tmr,* and *tml.* On the other hand TR can be totally dispensed with (M. De Beuckeleer *et al.* (1981) Molec. Gen. Genet. *183*:283-288, G. Ooms *et al.* (1982) Cell *30*:589-597), though found in a high copy number (D. J. Merlo *et al.* (1980) *supra*)*.* G. Ooms *et al.* (1982) Plasmid *7*:15-29, hypothesized that TR is involved in T-DNA integration, though they find that when TR is deleted from the Ti plasmid, *A. tumefaciens* does retain some virulence. G. Ooms *et al.* (1982) Cell *30*:589-597, showed that though T-DNA is occasionally deleted after integration in the plant genome, it is generally stable and that tumors containing a mixture of cells that differ in T-DNA organization are the result of multiple transformation events. The *ocs* is found in TL but can be deleted from the plant genome without loss of phenotypes related to tumorous growth. The left border of integrated TL has been observed to be composed of repeats of T-DNA sequences which are in either direct or inverted orientations (R. B. Simpson *et al.* (1982) Cell *29*:1005-1014).

In contrast to the situation in octopine-type tumors, nopaline T-DNA is integrated into the host genome in one continuous fragment (M. Lemmers *et al.* (1980) J. Mol. Biol. *144*:353-376, P. Zambryski *et al.* (1980) Science *209*:1385-1391). Direct tandem repeats were observed. T-DNA of plants regenerated from teratomas had minor modifications in the border fragments of the inserted DNA (Lemmers *et al., supra).* Sequence analysis of the junction between the right and left borders revealed a number of direct repeats and one inverted repeat. The latter spanned the junction (Zambryski *et al.* (1980) *supra).* The left junction has been shown to vary by at least 70 base pairs while the right junction varies no more than a single nucleotide (P. Zambryski *et al.* (1982) J. Molec. Appl. Genet. *1*:361-370). Left and right borders in junctions of tandem arrays were separated by spacers which could be over 130 bp. The spacers were of unknown origin and contained some T-DNA sequences. T-DNA was found to be integrated into both repeated and low copy number host sequences.

N. S. Yadav *et al.* (1982) Proc. Natl. Acad. Sci. USA *79*:6322-6326, have found a *chi* site, which in the bacteriophage λ augments general recombination in the surrounding DNA as far as 10 kilobases away, in a nopaline Ti plasmid just outside the left end of the T-DNA. R. B. Simpson *et al.* (1982) Cell *29*:1005-1014, have not observed a *chi* sequence in an octopine Ti plasmid, though the possible range of action does not eliminate the possibility of one being necessary and present but outside of the region sequenced. The significance of the *chi* in the Ti plasmid is not known. If the *chi* has a function, it is probably used in *Agrobacterium* cells and not in the plants, as *chi* is not found within the T-DNA.

### Agrobacterium-Manipulations of the TIP plasmids

As detailed in the section on Shuttle Vectors, technology has been developed for the introduction of altered DNA sequences into desired locations on a TIP plasmid. Transposons can be easily inserted using this technology (D. J. Garfinkel *et al.* (1981) Cell *27*:143-1531. J.-P. Hernalsteen *et al.* (1980) Nature *287*:654-456, have shown that a DNA sequence (here a bacterial transposon) inserted into T-DNA in the Ti plasmid is transferred and integrated into the recipient plant's genome. Though insertion of foreign DNA has been done with a number of genes from different sources, to date the genes have not been expressed under control of their own promoters. Sources of these genes include alcohol dehydrogenase (Adh) from yeast (K. A. Barton *et al.* (1983)); Adhl (J. Bennetzen, unpublished) and zein from corn, interferon and globin from mammals, and the mammalian virus SV40 (J. Schell, unpublished). M. Holsters *et al.* (1982) Mol. Gen. Genet. *185*:283-289, have shown that a bacterial transposon (Tn7) inserted into T-DNA could be recovered in a fully functional and seemingly unchanged form after integration into a plant genome.

Deletions can be generated in a TIP plasmid by several methods. Shuttle vectors can be used to introduce deletions constructed by standard recombinant DNA techniques (Cohen and Boyer US Pat. 4,237,224). Deletions with one predetermined end can be created by the improper excision of transposons (B. P. Koekman *et al.* (1979) Plasmid *2*:347-357, G. Ooms *et al.* (1982) Plasmid *7*:15-29). J. Hille and R. Schilperoot (1981) Plasmid *6*:151-154, have demonstrated that deletions having both ends at predetermined positions can be generated by use of two transposons. The technique can also be used to construct "recombinant DNA" molecules *in vivo.*

The nopaline synthetase gene has been used for insertion of DNA segments coding for drug resistance that can be used to select for transformed plant cells. M. Bevan (reported by M.D. Chilton *et al.* (18 January 1983) 15th Miami Winter Symp., see also J. L. Marx (1983) Science *219*:830) and R. Horsch *et al.* (18 January 1983) 15th Miami Winter Symp., see Marx, *supra,* have inserted the kanamycin resistance gene (neomycin phosphotransferase) from Tn5 behind (under control of) the nopaline promoter. The construction was used to transform plant cells which in culture displayed resistance to kanamycin and its analogs such as G418. J. Schell *et al.* (18 January 1983) 15th Miami Winter Symp. (see also Marx, *supra),* reported a similar construction, in which the methotrexate resistance gene (dihydrofolate reductase) from Tn7 was placed behind the nopaline synthetase promoter. Transformed cells were resistant to methotrexate. As plant cells containing octopine synthetase are resistant to the toxic chemical homo-arginine, G. M. S. Van Slogteren *et al.* (1982) Plant Mol. Biol. *1*:133-142, have proposed using that enzyme as a selectable marker.

M.-D. Chilton *et al.* (1983) *supra,* reported that A. Defremeux has constructed a "mini-Ti plasmid". In the nopaline T-DNA there is normally only one site cut by the restriction enzyme *Kpn*l. A mutant lacking the site was constructed and a Kpnl fragment, containing the entire nopaline T-DNA, was isolated. This fragment together with a kanamycin resistance gene was inserted into pRK290, thereby resulting in a plasmid which could be maintained in *A. tumefaciens* and lacked almost all non-T-DNA Ti sequences. By itself, this plasmid was not able to transform plant cells. However when placed in an *A. tumefacien* strain containing an octopine Ti plasmid, tumors were induced which synthesized both octopine and nopaline. This indicated that the missing nopaline Ti plasmid functions were complemented by the octopine Ti plasmid, and that the nopaline "mini-Ti" was functional in the transformation of plant cells. Chilton *et al.* (1983) *supra* also reported on the construction of a "micro-Ti" plasmid made by resectioning the mini-Ti with *Sma*l to delete essentially all of T-DNA but the nopaline synthetase gene and the left and right borders. The micro-Ti was inserted into a modified pRK290 plasmid that was missing its *Sma*l site, and employed in a manner similar to mini-Ti, with comparable results.

H. Lorz *et al.* (1982) in *Plant Tissue Culture 1982,* ed: A. Fujiwara, pp. 511-512, reported the construction of a plasmid vector, apparently independent of the TIP system of DNA uptake and maintenance, that used the nopaline synthetase gene as a marker.

### Phaseolin and gene regulation

In general the genes of higher eukaryotes are highly regulated. A multicellular organism, such as a plant has a number of differentiated tissues, each with its own specialized functions, each of which requires specialized gene products. One such tissue is the cotyledon. In legumes, the cotyledons usually serve as the storage tissue for the seed, holding reserves of lipid, carbohydrate, minerals, and protein until the seed needs them during germination. In *Phaseolus vulgaris* L. (also known as the French bean, kidney bean. navy bean, green bean and other names), the major storage protein is known as phaseolin. This protein comprises a small number of molecular species that are extremely homologous and equivalent to one another. Phaseolin contributes most of the nutrition value of dried beans, often comprising more than 10% of the weight of a dried bean.

Phaseolin is highly regulated during the life cycle of *P. vulgaris.* The protein is made essentially only while seed is developing within the pod. Levels rise from the limit of detection to as much as half the seed's protein content, following genetically determined schedules for synthesis. At its peak, phaseoiin synthesis can account for over 80% of a cotyledon cell's protein synthesis. At other times and in other tissues, phaseolin synthesis is undetectable. The extreme nature of phaseolin's regulation, coupled with its worldwide nutritional importance, has lead to much interest in the study of phaseoiin, its properties, and its regulation.

### Summary of the invention

The invention disclosed herein provides a plant comprising a genetically modified plant cell having a plant gene introduced and expressed therein. Further, the invention includes plant tissue comprising a plant cell whose genome includes T-DNA comprising a plant gene, said plant gene being expressible in the plant cell.

The experimental work disclosed herein is believed to be the first demonstration that plant genes are expressibie in plant cells after introduction via T-DNA, that is to say, by inserting the plant genes into T-DNA and introducing the T-DNA containing the insert into a plant cell using known means. The disclosed experiments are also believed to provide the first demonstration that plant genes containing introns are expressed in plant cells after introduction via T-DNA. These results are surprising in view of the fact that all genes previously known to be expressibie in T-DNA, either endogenous T-DNA genes or inserted foreign genes, lacked introns so far as is presently known. The results are unexpected also in view of the fact that the art has never previously been able to demonstrate that a promoter exogenous to the genes of T-DNA could function to control expression in a plant when introduced into T-DNA.

The invention is useful for genetically modifying plant tissues and whole plants by introducing useful plant genes from other plant species or strains. Such useful plant genes include, but are not limited to, genes coding for storage proteins, lectins, resistance factors against disease, insects and herbicides, factors providing tolerance to environmental stress, genes for specific flavor elements, and the like. The invention is exemplified by the introduction and expression of phaseolin, a major seed storage protein of beans, into sunflower and tobacco plant tissues. Once plant cells expressing a plant gene introduced via T-DNA are obtained, plant tissues and whole plants can be regenerated therefrom by means of methods known in the art. The regenerated plants are then reproduced by conventional means and the introduced genes can be transferred to other strains by conventional plant breeding techniques. The introduction and expression of the phaseolin gene, for example, can be used to enhance the protein content and nutritional value of forage crops such as alfalfa. Other uses of the invention, exploiting the properties of other genes, introduced into other plant species, will be readily apparent to those skilled in the art. The invention in principle applies to any introduction of plant genes into any plant species into which T-DNA can be introduced and in which T-DNA can remain stably replicated. In general these species include, but are not limited to, dicotyledonous plants, such as sunflower (family *Compositeae*), tobacco (family *Solanaceae*), alfalfa, soybeans and other legumes (family *Legumincseae*) and most vegetables.

### Detailed description of the invention

The following definitions are provided, in order to remove ambiguity as to the intent or scope of their useage in the specification and claims.

T-DNA: A segment of DNA derived from the transformation-inducing principle (TIP) which becomes integrated in the plant genome. As used herein, the term includes DNA originally derived from any tumor inducing strain of *Agrobacterium* including *A. tumefaciens* and *A. rhizogenes,* the latter sometimes referred to in the prior art as R-DNA. In addition, as used herein the term T-DNA includes any alterations, modifications, mutations, insertions and deletions, either naturally occurring or introduced by laboratory procedures, the only structural requirement being that sufficient to the right and left ends of naturally occurring T-DNAs be present to ensure the expected function of stable integration which is characteristic of all T-DNA.

plant gene: As used herein includes both structural and regulatory elements of a plant gene said elements being exogenous to the genes of T-DNA itself. As used herein, a plant gene is one in which both the promoter (a region of the gene which provides for and may regulate the initiation of transcription and the initiation of translation) and the structural gene (the region which codes for a protein and which may or may not contain one or more introns) are introduced into T-DNA from a plant source. The plant gene may also include a 3'-untranslated region which may function to regulate termination of transcription, and post-transcriptional RNA processing. The promoter and structural gene elements may be derived from the same, or different pre-existing genes and may be derived from the same or different plant sources. For example, a plant gene could be, as exemplified herein, a plant gene with its own promoter, or it could be an *in vitro* construct comprising the coding region (with or without introns) of one gene and the promoter of another, derived from the same or different plant species. The coding region of a plant gene, as herein defined, may include a cDNA copy of the structural portion of a plant gene. The promoter and coding regions may also include modifications, either naturally or artificially induced, and may include chemically synthesized segments. The coding segment may itself be a composite of segments derived from a plurality of sources, naturally occurring or synthetic, coding for a composite protein.

plant tissue: Includes differentiated and undifferentiated tissues of, and derived from higher plants including roots, shoots, pollen, seeds, tumor tissue, such as crown galls, and various forms of aggregations of plant cells in culture, such as embryos and calluses.

plant cell: As used herein includes plant cells and protoplasts in culture.

Production of a genetically modified plant expressing a plant gene introduced via T-DNA combines the specific teachings of the present disclosure with a variety of techniques and expedients known in the art. In most instances, alternative expedients exist for each stage of the overall process. The choice of expedients depends on variables such as the choice of the basic TIP, the plant species to be modified and the desired regeneration strategy, all of which present alternative process steps which those of ordinary skill are able to select and use to achieve a desired result. The fundamental aspects of the invention are the nature and structure of the plant gene and its means of insertion into T-DNA. The remaining steps to obtaining a genetically modified plant include transferring the modified T-DNA to a plant cell wherein the modified T-DNA becomes stably integrated as part of the plant cell genome, techniques for *in vitro* culture and eventual regeneration into whole plants, which may include steps for selecting and detecting transformed plant cells and steps of transferring the introduced gene from the originally transformed strain into commercially acceptable cultivars.

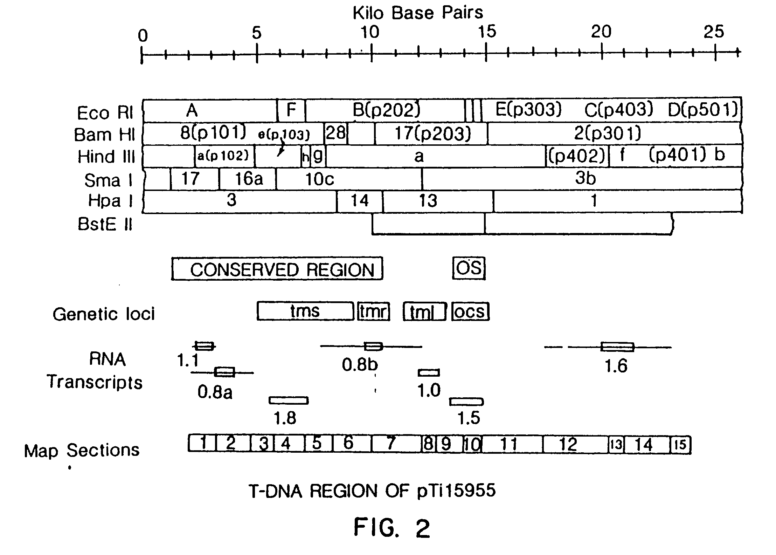

A principal feature of the present invention is the construction of T-DNA having an inserted plant gene, as defined *supra.* Location of the plant gene insertion site is not critical as long as the transfer function of sequences immediately surrounding the T-DNA borders are not disrupted, since these regions appear from prior art studies to be essential for insertion of the modified T-DNA into the plant genome. Preferred insertion sites are those which lie in areas that are most actively transcribed, in particular the *tml* gene and an area designated "1.6" lying in the *Hind*III-f fragment, and spanning map section 13, as shown in Fig. 2. No phenotype has been associated with the latter transcript. The term "1.6" is used herein to designate this actively transcribed region of T-DNA. T-DNA is obtained from any of the TIP plasmids. The plant gene is inserted by standard techniques well known to those skilled in the art. The orientation of the inserted plant gene, with respect to the direction of transcription and translation of endogenous T-DNA genes is not critical, either of the two possible orientations is functional. Differences in rates of expression may be observed when a given gene is inserted at different locations within T-DNA, possibly because of such factors as chromatin structure. Readily detectable levels of expression of the phaseolin gene have been obtained where that gene was inserted in a *Sma*l site within the *tml* gene of pTi15955, an octopine-type plasmid of *A. tumefaciens.*

A convenient means for inserting a plant gene into T-DNA involves the use of shuttle vectors, as described *supra,* having a segment of T-DNA (that segment into which insertion is desired) incorporated into a plasmid capable of replicating in *E. coli.* The T-DNA segment contains a restriction site, preferably one which is unique to the shuttle vector. The plant gene can be inserted at the unique site in the T-DNA segment and the shuttle vector is transferred into cells of the appropriate *Agrobacterium* strain, preferably one whose T-DNA is homologous with the T-DNA segment of the shuttle vector. The transformed *Agrobacterium* strain is grown under conditions which permit selection of a double homologous recombination event which results in replacement of a pre-existing segment of the Ti plasmid with a segment of T-DNA of the shuttle vector.

Following the strategy just described, the modified T-DNA can be transferred to plant cells by any technique known in the art. For example, this transfer is most conveniently accomplished either by direct infection of plants with the novel *Agrobacterium* strain containing a plant gene incorporated within its T-DNA, or by co-cultivation of the *Agrobacterium* strain with plant cells. The former technique, direct infection, results in due course in the appearance of a tumor mass or crown gall at the site of infection. Crown gall cells can be subsequently grown in culture and, under appropriate circumstances known to those of ordinary skill in the art, regenerated into whole plants that contain the inserted T-DNA segment. Using the method of co-cultivation, a certain proportion of the plant cells are transformed, that is to say have T-DNA transferred therein and inserted in the plant cell genome. In either case, the transformed cells must be selected or screened to distinguish them from untransformed cells. Selection is most readily accomplished by providing a selectable marker incorporated into the T-DNA in addition to the plant gene. Examples include either dihydrofolate reductase or neomycin phosphotransferase expressed under control of a nopaline synthetase promoter. These markers are selected by growth in medium containing methotrexate or kanamycin, respectively, or their analogs. In addition, the T-DNA provides endogenous markers such as the gene or genes controlling hormone-independent growth of Ti-induced tumors in culture, the gene or genes controlling abnormal morphology of Ri-induced tumor roots, and genes that control resistance to toxic compounds such as amino acid analogs, such resistance being provided by an opine synthetase. Screening methods well known to those skilled in the art include assays for opine production, specific hybridization to characteristic RNA or T-DNA sequences, or immunological assays for specific proteins, including ELISA, (acronym for "Enzyme Linked Immunosorbent Assay") radioimmune assays and "western" blots.

An alternative to the shuttle vector strategy involves the use of plasmids comprising T-DNA or modified T-DNA, into which a plant gene is inserted, said plasmids being capable of independent replication in an *Agrobacterium* strain. Recent evidence indicates that the T-DNA of such plasmids can be transferred from an *Agrobacterium* strain to a plant cell provided the *Agrobacterium* strain contains certain trans-acting genes whose function is to promote the transfer of T-DNA to a plant cell. Plasmids that contain T-DNA and are able to replicate independently in an *Agrobacterium* strain are herein termed "sub-TIP" plasmids. A spectrum of variations is possible in which the sub-TIP plasmids differ in the amount of T-DNA they contain. One end of the spectrum retains all of the T-DNA from the TIP plasmid, and is sometimes termed a "mini-TIP" plasmid. At the other end of the spectrum, all but the minimum amount of DNA surrounding the T-DNA border is deleted, the remaining portions being the minimum necessary to be transferrable and integratable in the host cell. Such plasmids are termed "micro-TIP". Sub-TIP plasmids are advantageous in that they are small and relatively easy to manipulate directly. After the desired gene has been inserted, they can easily be introduced directly into a plant cell containing the trans-acting genes that promote T-DNA transfer. Introduction into an *Agrobacterium* strain is conveniently accomplished either by transformation of the *Agrobacterium* strain or by conjugal transfer from a donor bacterial cell, the techniques for which are well known to those of ordinary skill.

Regeneration is accomplished by resort to known techniques. An object of the regeneration step is to obtain a whole plant that grows and reproduces normally but which retains integrated T-DNA. The techniques of regeneration vary somewhat according to principles known in the art, depending upon the origin of the T-DNA, the nature of any modifications thereto and the species of the transformed plant. Plant cells transformed by an Ri-type T-DNA are readily regenerated, using techniques well known to those of ordinary skill, without undue experimentation. Plant cells transformed by Ti-type T-DNA can be regenerated, in some instances, by the proper manipulation of hormone levels in culture. Preferably, however, the Ti-transformed tissue is most easily regenerated if the T-DNA has been mutated in one or both of the Tmr and Tms genes. Inactivation of these genes returns the hormone balance in the transformed tissue towards normal and greatly expands the ease and manipulation of the tissue's hormone levels in culture, leading to a plant with a more normal hormone physiology that is readily regenerated. In some instances, tumor cells are able to regenerate shoots which carry integrated T-DNA and express T-DNA genes, such as nopaline synthetase, and which also express an inserted plant gene. The shoots can be maintained vegetatively by grafting to rooted plants and can develop fertile flowers. The shoots thus serve as parental plant material for normal progeny plants carrying T-DNA and expressing the plant gene inserted therein.

The genotype of the plant tissue transformed is often chosen for the ease with which its cells can be grown and regenerated in *in vitro* culture. Should a cultivar of agronomic interest be unsuitable for these manipulations, a more amenable variety is first transformed. After regeneration, the newly introduced foreign plant gene is readily transferred to the desired agronomic cultivar by techniques well known to those skilled in the arts of plant breeding and plant genetics. Sexual crosses of transformed plants with the agronomic cultivars yielded initial hybrid. These hybrids can then be back crossed with plants of the desired genetic background. Progeny are continuously screened and selected for the continued presence of integrated T-DNA or for the new phenotype resulting from expression of the inserted plant gene. In this manner, plants can be produced having a genotype essentially identical to the agronomically desired parents with the addition of the inserted plant gene, after a number of rounds of back crossing and selection.

### Examples

The following Examples utilize many techniques well known and accessible to those skilled in the arts of molecular biology and manipulation of TIPs and *Agrobacterium;* such methods are not always described in detail. Enzymes are obtained from commercial sources and are used according to the vendor's recommendations or other variations known to the art. Reagents, buffers and culture conditions are also known to those in the art. Reference works containing such standard techniques include the following: R. Wu, ed. (1979) Meth. Enzymol, 68; J. H. Miller (1972) *Experiments in Molecular Genetics; R.* Davis *et al.* (1980) *Advanced Bacterial Genetics;* and R. F. Schleif and P. C. Wnesink (1982) *Practical Methods in Molecular Biology.*

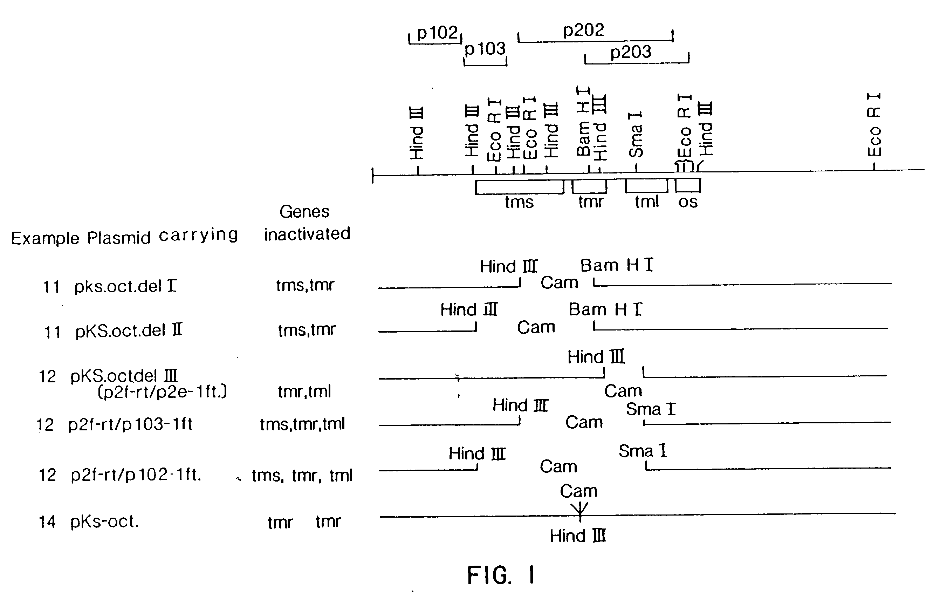

With the exception of the plasmid IIc, plasmids, and only plasmids, are prefaced with a "p", e.g., p3.8 or pKS4. Cells containing plasmids are indicated by identifying the cell and parenthetically indicating the plasmid. *e.g., A. tumefaciens* (pTi15955) or K802(pKS4-KB). Table 1 provides an index useful for identifying plasmids and their interrelationships. Table 2 provides an index of deposited strains. Fig. 1 provides a useful comparison of the constructions described in Examples 5, 6 and 8.

### Example 1

The purpose of this example is to teach the expression of a non-T-DNA eukaryotic gene under control of its own promoter.

### 1.1 Preparation of special plasmid derivatives

A restriction site was removed from the plasmid pBR322 by digestion with *Hin*dIII filling in the single-stranded sticky-ends by DNA polymerase I, blunt-end ligation, transformation into K802, selection for tetracycline resistance, plasmid isolation from the drug resistant clones, and characterization with restriction enzymes to confirm elimination of the appropriate restriction site. The plasmid was labeled p350 (pBR322-*Hin*dIII).

### 1.2 Preparation of shuttle vectors

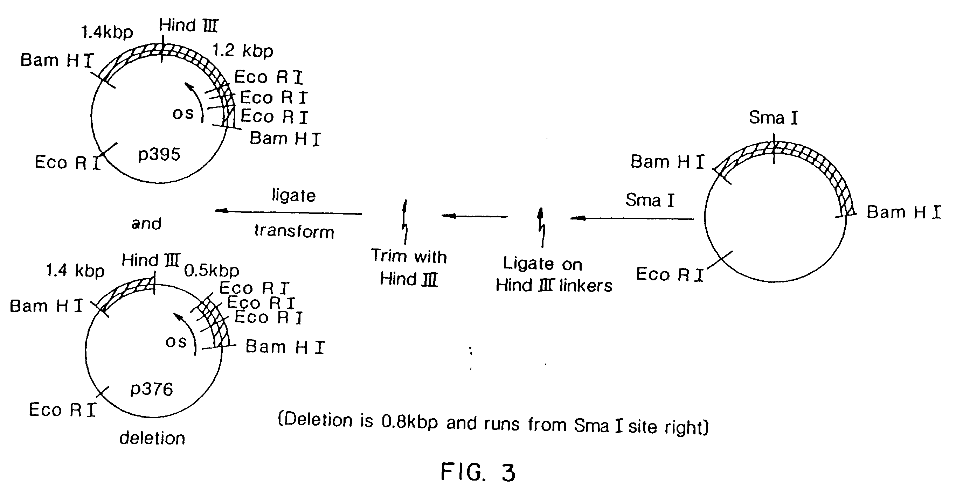

p203 was digested with *Bam*HI and the T-DNA Bam 17 fragment (see Fig. 2) was isolated by agarose gel electrophoresis followed by elution from the gel. This fragment was mixed with and ligated to *Bam*HI-linearized p350 and the reaction was transformed into K802. Plasmid isolated from ampicillin resistant transformants was characterized by restriction mapping, digested with *Sma*l*,* and blunt-end ligated to *Hin*dIII linkers. After the *Hin*dIII sticky-ends were unmasked by trimming with *Hin*dIII and the linearized plasmid was circularized by ligation to itself, the plasmid transformed into K802. Plasmid isolated from ampicillin resistant transformants was characterized by restriction mapping and a plasmid having a structure as shown in Fig. 3 was labeled p395. p376. to be discussed below, was also isolated at this point (Fig. 3).

p395 was digested with *Bam*HI*,* and the Bam 17 T-DNA fragment, which had had its *Sma*I site converted to a *Hind*lll site, was isolated by agarose gel electrophoresis followed by elution. This Bam 17 fragment was mixed with and ligated to *Bgl*II-linearized pRK290. The reaction mixture was transformed into K802, and after selection, transformants were used to prepare plasmids which were characterized by restriction mapping. The appropriate plasmid was labeled p490-8/14.

p376, whose derivation was described above in this example, was found to contain a deletion of about 0.8 kbp running to the right from the *Sma*I, now converted to the specificity of *Hind*III. As was done with p395 above, the *Bam*HI T-DNA fragment corresponding to Bam 17 was isolated and spliced into *Bgl*II-linearized pRK290. After transformation, selection, and plasmid isolation and characterization, the appropriate plasmid was labeled p458-1.

### 1.3 Insertion of the kanamycin resistance and phaseolin genes

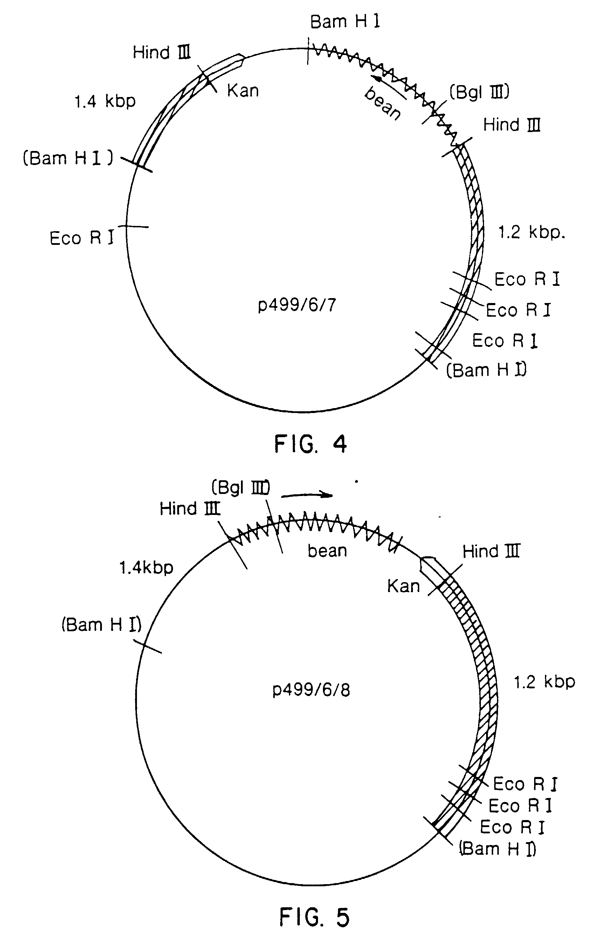

The phaseolin gene-carrying fragment was purified from *Hind*III digested pKS-KB3.8 by agarose gel electrophoresis. This fragment was mixed with and ligated to *Hind*III-linearized p490-8/14. Kanamycin resistant transformants of K802 were used to prepare plasmids which were then restriction mapped. Two constructions were isolated: p499/6/7 had bean sequences to the right of the kanamycin resistance (Fig. 4), and p499/6/8 had the opposite orientation (Fig. 5).

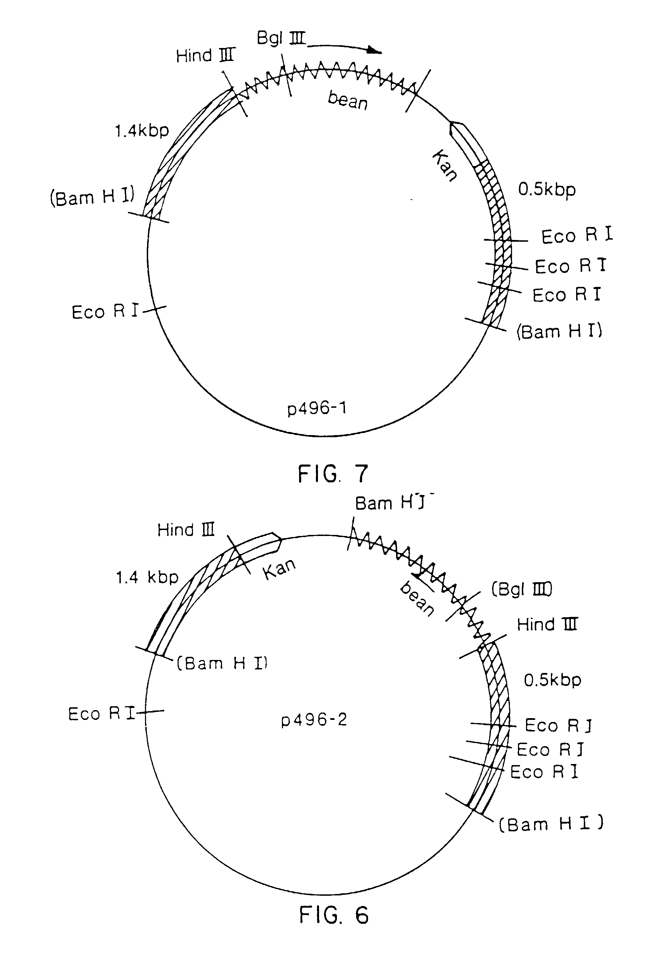

The purified *Hind*III fragment of pKS-KB3.8 carrying the phaseolin gene was also mixed with and ligated to *Hind*III-linearized p458-1. Plasmids were again prepared from kanamycin resistant transformants of K802 and restriction mapped. Again both orientations were isolated: p496-2 (Fig. 6) and p496-1 (Fig. 7) respectively had phaseolin to the right and left of the kanamycin resistance gene.

### 1.4 Double homologous recombination with Ti plasmids

The phaseolin and kanamycin genes were integrated into Ti plasmids harbored within *Agrobacterium tumefaciens* cells as described in Example 14. Two Ti plasmids were used as recipients: pTi15955, an octopine-type plasmid; and pTiA66, a strain derived from the A6 octopine-type plasmid which has a nonfunctional *tms* gene due to a natural insertion of an *Agrobacterium IS* (insertion) sequence. pTi15955 plasmids containing the constructions defined by p499/6/7, p499/6/8, p496-2, and p496-1, are respectively labeled p529-8, p529-7, p529-11 and p529-2. The same constructions in pTiA66 are respectively labeled p539-6, p539-5, p539-2, and p539-1.

### 1.5 Infection of plants

*A. tumefaciens* cells containing the Ti plasmids of the p529 and p539 series were used to infect stems of sunflower plants by puncture with a needle followed by injection of the appropriate bacterial cells.

### 1.6 Detection of phaseolin

Phaseolin protein sequences were detected to galls by ELISAs as described in Example 13. All galls tested were found to contain phaseolin; the level varied between 20 ng and 0 ng per gram tissue fresh weight, with the average being about 10 ng/g. Analysis by western blots of denaturing protein gels (SDS-polyacrylamide) showed discrete bands, of high apparent molecular weight, though significantly smaller than native phaseolin. The exact number and sizes of the bands varied between host plants, indicating that they were the results of host specific post-translational processing.

Phaseolin mRNA sequences were detected in galls as described in Example 12. All galls tested were found to contain phaseolin sequences in the poly(A)5+4RNA fraction; the level averaged about 0.005% of total poly(A)RNA. Analysis by northern blots of denaturing DNA gels (methyl mercury-agarose) showed a descrete band of high molecular weight the same size as natural phaseolin mRNA (1.6 kbp).

Phaseolin was also detected by ELISA in shoot tissue derived from cells infected with a pTiA66-based vector.

Detection levels of both phaseolin protein and phaseolin mRNA signals were significantly and substantially above the noise levels found when assaying galls transformed by *A. tumefaciens* cells harboring unmodified pTi15955 and unmodified pTiA66.

### Example 2

This example teaches the insertion of complete phaseolin gene into T-DNA analogous to that taught in Example 1. This construction utilizes a shuttle vector designed to carry inserted sequences into a nopaline Ti plasmid, pTiC58, in the region of the nopaline synthetase gene.

### 2.1 Construction of a shuttle vector

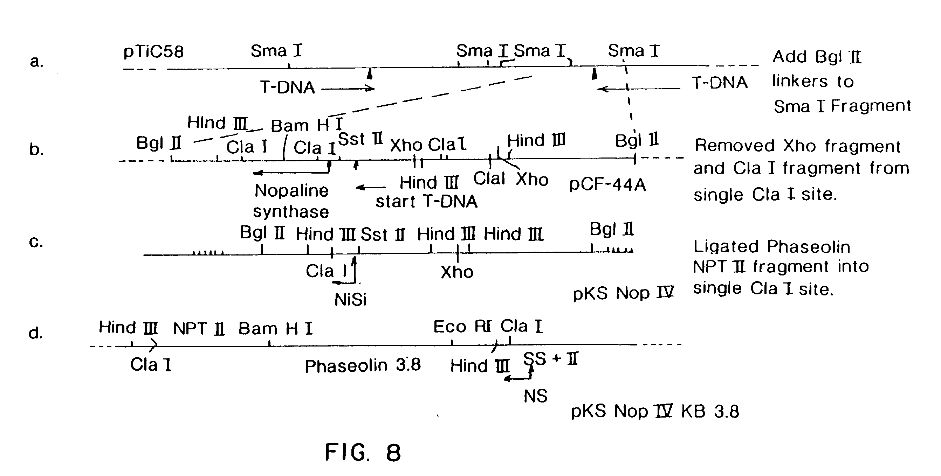

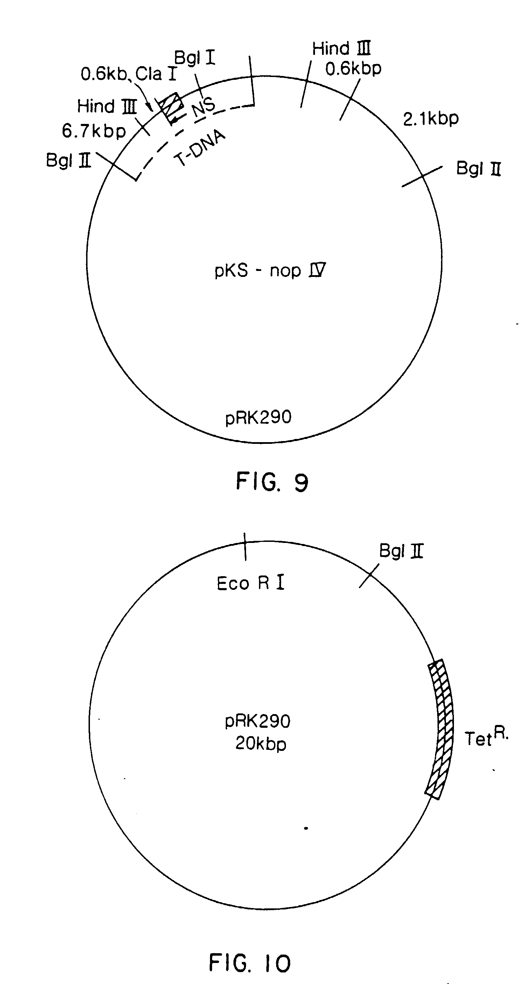

The nopaline-type plasmid, pTiC58 (Fig. 8a) was digested with *Sma*l*,* and a fragment encoding the nopaline synthetase gene was isolated by agarose gel electrophoresis. This fragment was blunt-end ligated to *Bgl*II linkers, which were then unmasked by digestion with *Bgl*II. The resulting DNA fragment was mixed with and ligated to *Bgl*II-linearized pRK290 (Fig. 10). Transformation into K802 was followed by selection with tetracycline, plasmid isolation, and restriction mapping. The appropriate plasmid was labeled pCF44A (Fig. 8b).

The four *Cla*I sites were reduced to a single *Cla*I susceptible site by serially resectioning pCF44A twice. The plasmid was digested with *Xho*I*,* religated to itself, and transformed into K802. After tetracycline selection, plasmid isolation, and restriction mapping, the appropriate plasmid having a deletion of an *Xhol* fragment which carries two *Cla*I sites was digested with *Cla*I, religated to itself, and transformed into K802. After selection, plasmid isolation, and restriction mapping the appropriate plasmid having a second deletion, this time of the *Cla*I fragment which carries all but the 5' end of the *nos* gene, was labeled pKS-nopIV (Figs. 9, 8, 8c).

### 2.2 Insertion of the kan/bean genes

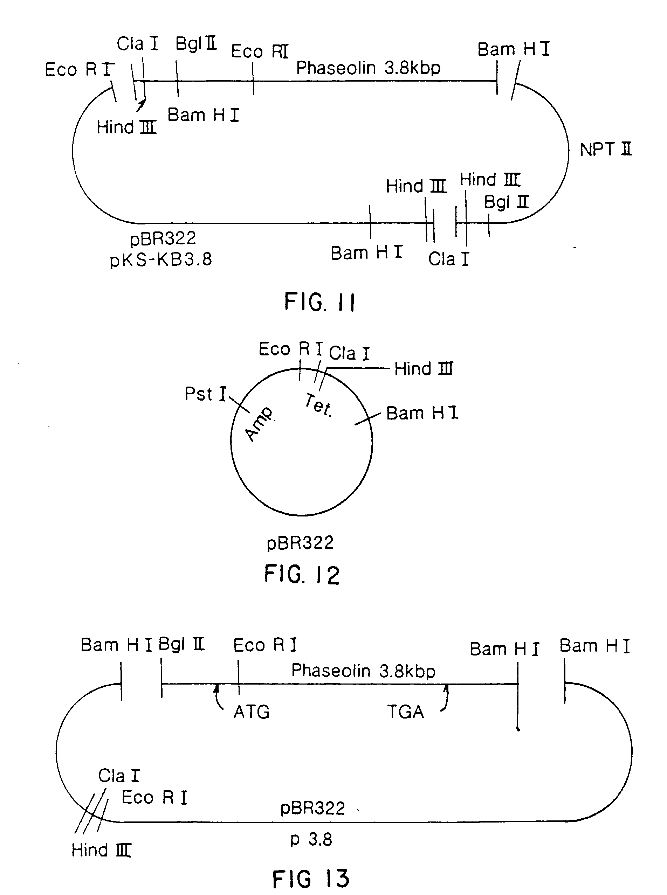

pKS-KB3.8 (Fig. 11) was digested with *Cla*I*,* and the 6.0 kbp fragment carrying the kanamycin resistance and phaseolin genes was isolated by agarose gel electrophoresis. This fragment was mixed with and ligated to *Cla*l-linearized pKS-noplV, and transformed into K802. Plasmids isolated from transformants resistant to kanamycin and tetracycline and sensitive to ampicillin were restriction mapped and one having the structure shown in Fig. 8d was labeled pKS-nopIV-KB3.8#5. A similar clone oriented with the phaseolin gene to the left of the kanamycin resistance was found and labeled pKS-nopKB3.8#3.

### 2.3 Transfer to Ti plasmids and infection of plants

The triparental mating technique (see Background and Example 14) was used to transfer the constructions to pTiC58, a nopaline-type Ti -plasmid. Ti plasmids C58-nopKB#3 and pC58-nop-KB#5, the results of matings of pKS-noplV-KB3.8#3 and #5, respectively, with pTiC58 were characterized by restriction site mapping and Southern blot analysis. Bacteria containing either of the two plasmids, having either orientation of the kanamycin resistance/phaseolin gene fragment nested within sequences from the 5' end of the *nos* gene and the *nos* gene's 3' flanking sequences, were used separately to infect stems of sunflower plants by puncture followed by injection of bacteria.

### 2.4 Detection of expression

Phaseolin gene expression was detected in sunflower gall tissue by ELISAs, as in Example 13.5

### Example 3

This example teaches manipulations of a gene for phaseolin, the major seed storage protein of the bean *Phaseolus vulgaris* L., preparatory to further manipulations which insert the phaseolin gene into vectors described in various other examples.

### 3.1 Subcloning of a phaseolin gene

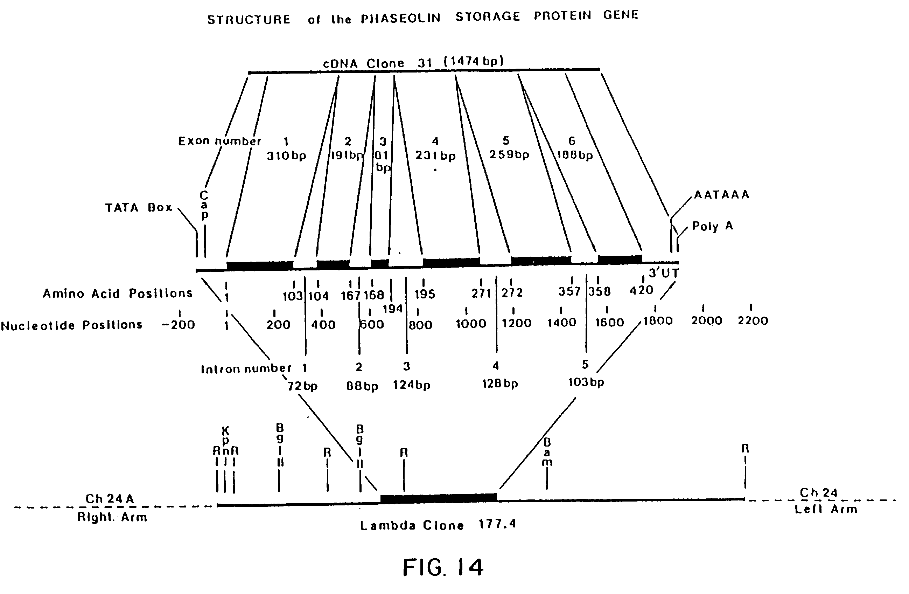

A genomic clone of phaseolin in a Charon 24A AG-PVPh177.4 (or 177.4; S. M. Sun *et al,* (1981) Nature *289*:37-41, J. L. Slightom *et al*. (1983) Proc. Natl. Acad. Sci. USA *80*; Fig. 14 was digested wit *Bgl*II and *Bam*HI*.* The 3.8kbp fragment carrying the phaseolin gene and its flanking sequences, isolated by agarose gel electrophoresis, was mixed with and ligated to *Bam*HI-linearized pBR322 (Fig. 12). The mixture was transformed into HB101, and colonies resistant to ampicillin and sensitive to tetracycline were selected. Plasmid isolated from these clones was restriction mapped. A plasmid having the structure shown in Fig. 13 was selected and labeled AG-pPVPh3.8 (or alternatively, p3.8). The ligation of *Bgl*II and *Bam*HI sites with each other inactivates both sites.

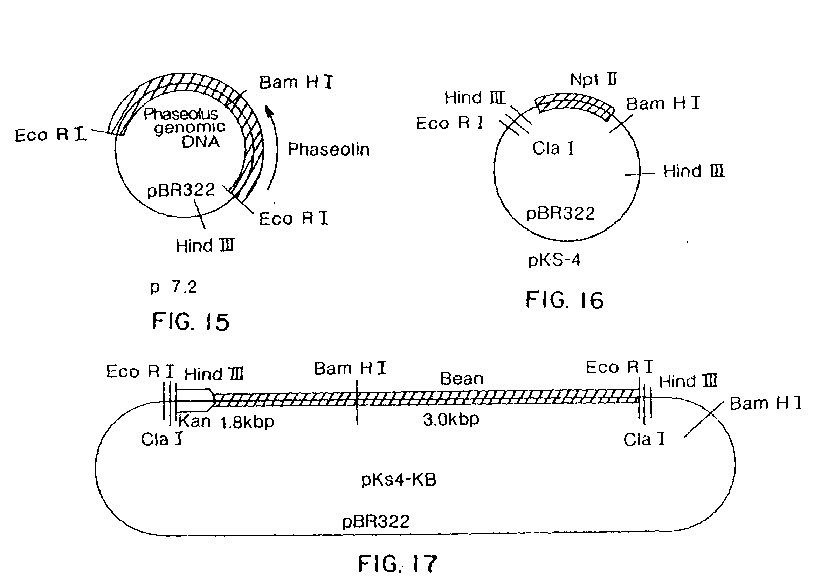

Another subclone of 177.4 was constructed by digestion with *Eco*RI*,* isolation of a 7.2 kbp fragment containing extensive 3' flanking sequences and all but the extreme 5' end of the phaseolin gene, and isolated after ampicillin selection of HB101 transformants were restriction mapped. A plasmid having the insert oriented so that the *Hind*lll site of pBR322 was adjacent to the 5' end of the phaseolin gene and distal to the 3' untranslated region was labeled AG-pPVPh7.2 (or p7.2; Fig. 15; Sun *et al.* and Slightom *et al., supra).*

### 3.2 Cloning and isolation of a kanamycin resistance gene

pRZ102 (R. A. Jorgenson *et al.* (1979) Mol. gen. Genet. 177:65-72), a ColE1 plasmid carrying a copy of the transposon Tn5, was digested with *Bam*HI and *Hind*III, mixed with pBR322 previously linearized with the same two enzymes, ligated, and transformed into K802. Plasmids, isolated from transformants selected for resistance to both ampicillin and kanamycin were restriction mapped and one having the structure shown in Fig. 16 was labeled pKS-4.

### 3.3 Linkage of the phaseolin gene with a kanamycin resistance

p3.8 was digested with *Cla*I and *Bam*HI*,* and a 4.2kbp fragment containing the phaseolin gene and some pBR322 sequences was isolated by agarose gel electrophoresis. This was mixed with a *Cla*I*/Bam*HI fragment of Tn5 carrying a kanamycin resistance (neomycin phosphotransferase II, NPTII) gene from pKS4 (Fig. 16) and pBR322 (Fig. 12) which had been linearized with Clal. The mixture was ligated and transformed into K802. After selection of colonies resistant to ampicillin and kanamycin, plasmids were isolated and restriction mapped. A colony having the structure shown in Fig. 11 was labeled pKS-KB3.8.

p7.2 was digested with *EcoRI* and *Bam*HI, and a 3.0kbp fragment carrying all but the 5' end of the phaseolin gene was isolated by agarose gel electrophoresis. This was mixed with a *Hin*dIII*/Bam*HI fragment of Tn5 carrying a kanamycin resistance gene from pKS4 (Fig. 16) and pBR322 (Fig. 12) which had been linearized with Hindlll. The mixture was ligated and transformed into K802. After selection of colonies resistant to ampicillin and kanamycin, plasmids were isolated and restriction mapped. A colony having the structure shown in Fig. 17 was labeled pKS4-KB3. In pKS4-KB, phaseolin is missing sequences encoding the extreme 5' end of the gene, and all 5' flanking regions (see Fig. 14).

### Example 4

This example teaches a method of removing the introns from a gene. This is the same as placing a cDNA in a genomic environment. Restriction enzyme sites are found, or created by site specific mutagenesis, in exons on both the 5' and 3' extremities of the unprocessed transcript. These sites exist in both the genomic clones and cDNA. The intervening intron-containing DNA can be removed from the genomic clone and be replaced with the corresponding intronless cDNA clone fragment spanning the two sites. The reverse operation is also possible: intron-containing genomic sequences can be placed in a cDNA environment. One inserts an internal fragment of the genomic clone into a corresponding gap cut out of a cDNA clone. This latter strategy is analogous, though often technically more difficult as the introns may contain sites susceptible to the enzymes chosen to create the exchanged fragment. This difficulty can be overcome by careful selection of conditions of partial digestion and by purification of the desired fragment by agarose gel electrophoresis. Further elaborations of this strategy include the manipulation of individual introns within a gene while leaving other introns and exons unaffected, and the stepwise exchange of sequences when inconvenient intervening restriction sites are present within introns as discussed above.

### 4.1 Replacement of a fragment containing phaseolin's introns with cDNA

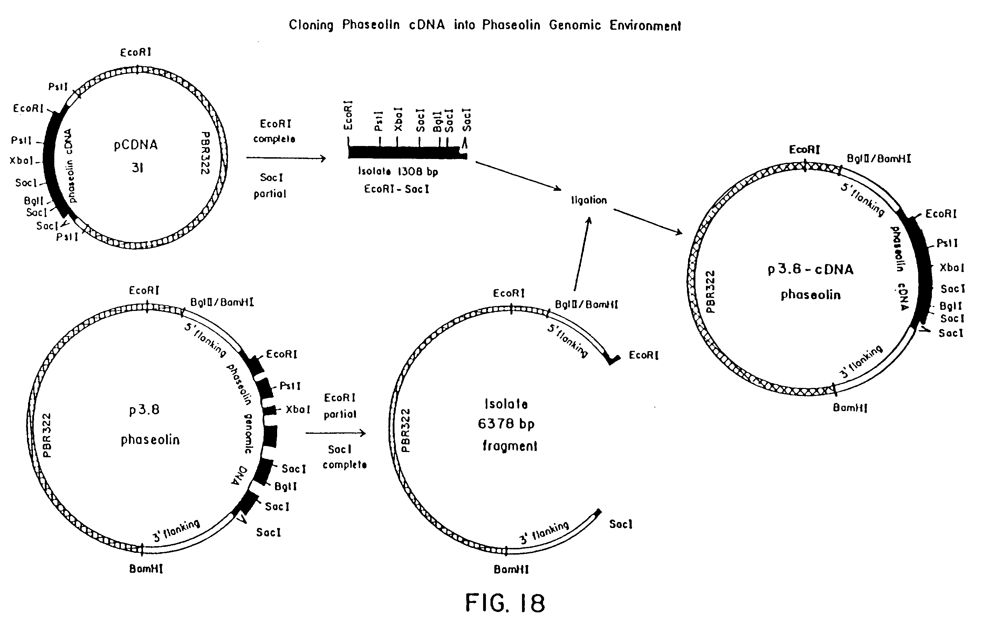

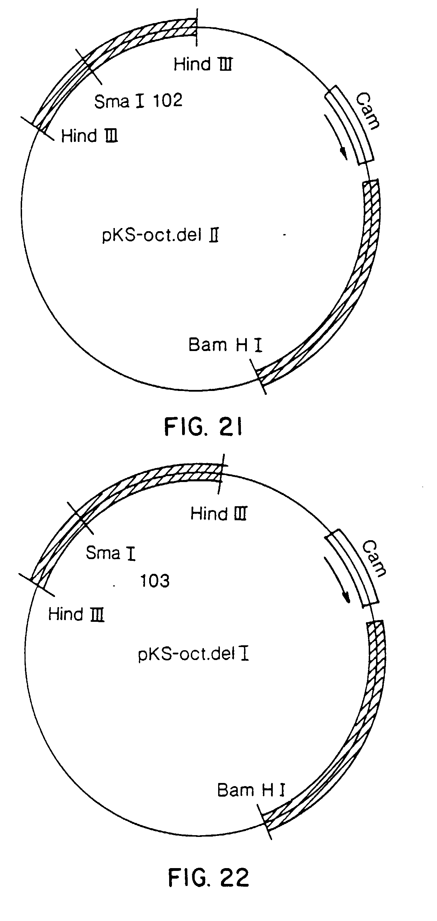

p3.8, a plasmid clone of the phaseolin gene and its flanking sequences, was digested respectively partially and to completion with *Eco*RI and *Sac*I*,* and a 6.4kbp fragment, containing the pBR322 vector and both the 5' and 3' ends of the gene, was isolated by agarose gel electrophoresis, pcDNA31, a pBR322 plasmid clone of cDNA made from phaseolin mRNA, was digested respectively partially and to completion with *Sac*I and *Eco*RI*,* and a 1.33kbp fragment, containing the entire phaseolin cDNA except for sequences at the extreme 5' and 3' ends, was isolated by agarose gel electrophoresis. These two fragments were ligated together and transformed into HB101. After selection of colonies, growth of cells, and plasmid isolation, restriction mapping identified a plasmid having the desired structure. This plasmid was labeled p3.8-cDNA (Fig. 22). The entire construction is diagrammed in Fig. 18.

### 4.2 Use of p3.8-cDNA

Note that p3.8-cDNA can substitute for the genomic DNA source, e.g., p3.8, used in all other Examples, and that when so used will result in analogous constructions differing in that they are lacking introns. Alternatively, this strategy can be used to remove introns from constructions already made.

### Example 5

The purpose of this example is to generate a Ti plasmid with a deletion from the *tms* ("shooting" locus) through the *tmr* ("rooting" locus) of pTi159 and other octopine Ti plasmids. This derivative is useful because cells transformed by it are easier to regenerate to whole plants than cells transformed by pTi15955 with intact *tms* and *tmr* genes.

The *tms-tmr* deleted pTi15955 is ultimated changed in two ways: the inactivation of *tms-tmr* and the insertion of a foreign gene. Should these two changes be located at different points of the T-DNA, each change is inserted independently by different shuttle vectors. Each shuttle vector dependent change is selected independently which will necessitate use of at least two markers selectable in *Agrobacterium.* In addition to the usual kanamycin resistance, this example utilized a chloramphenicol resistance derived from pBR325.

### 5.1 Construction of a chloramphenicol resistance gene clone

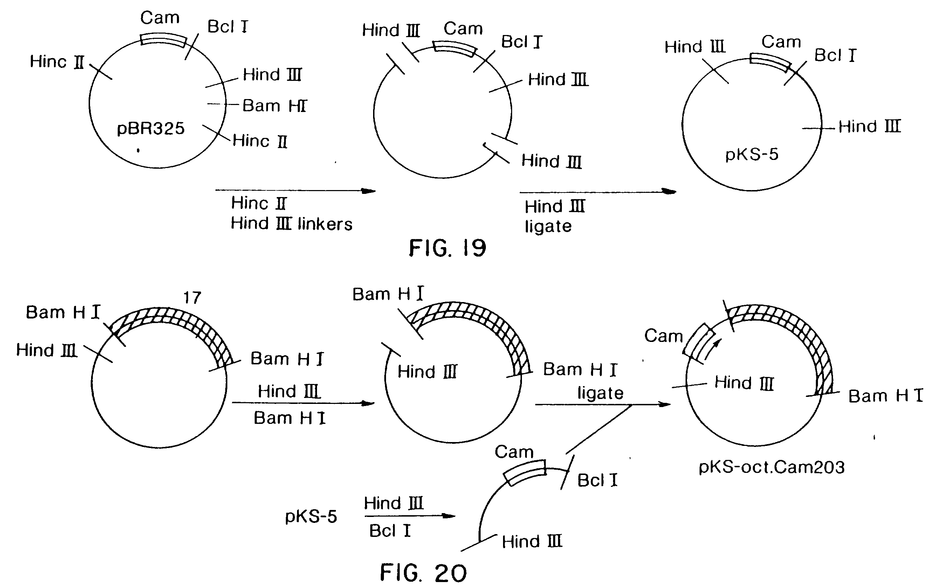

pBR325 is digested with *Hin*cII and blunt end ligated with *Hin*dIII linkers. The resultant preparation is digested with *Hin*dIII, religated, selected for chloramphenicol resistance *(cam),* and labeled pKS-5 which will serve as a source of the *Hin*dIII/*Bcl*I fragment which contains the *cam* gene (Fig. 19).

### 5.2 Construction of a pBR322 clone of T-DNA with a deletion and a cam gene

A 9.2kbp linear DNA fragment is isolated from a complete *Hind*III and partial *Bam*HI digest of p203 (Fig. 31). The fragment carrying the *cam* gene is isolated from pKS-5, mixed with the 9.2kbp linear fragment, ligated, transformed into *E. coli,* selected for chloramphenicol resistance, and labeled pKS-oct.Cam203 (Fig. 20).

pKS-oct.Cam203 is a plasmid clone that can now be used to construct a number of deletion TL mutants of pTi15955. It contains the right hand arm of TL and a resistance gene to the left of the right arm. Various left-hand arms of TL can be attached to the left of the *cam* gene (*Hind*III site). For instance, if p102 is attached the deletion is 5.2kbp long and includes all of *tms* and *tmr.* If p103 is attached the deletion is 3.2kbp long and includes part of *tms* and all of *tmr.* See Fig. 2.

pKS-oct.Cam203 is digested with *Hin*dIII. p102 or p103 is digested with *Hin*dIII and the 2.2kbp or 2.0kbp T-DNA fragment is isolated and ligated with the linearized pKS-oct.Cam203, transformed, isolated yielding pKS-oct.delll (Fig. 21) or pKS-oct.dell (Fig. 22) respectively. These constructions are moved into *A. tumefaciens* by mating, homologous recombinations, and selection for chloramphenicol resistance. Alternatively, one moves the constructions into pRK290 by use of established methods by linearizing the construction carrying plasmids with *Bam*HI and ligating into the *Bgl*II site of pRK290.

### Example 6

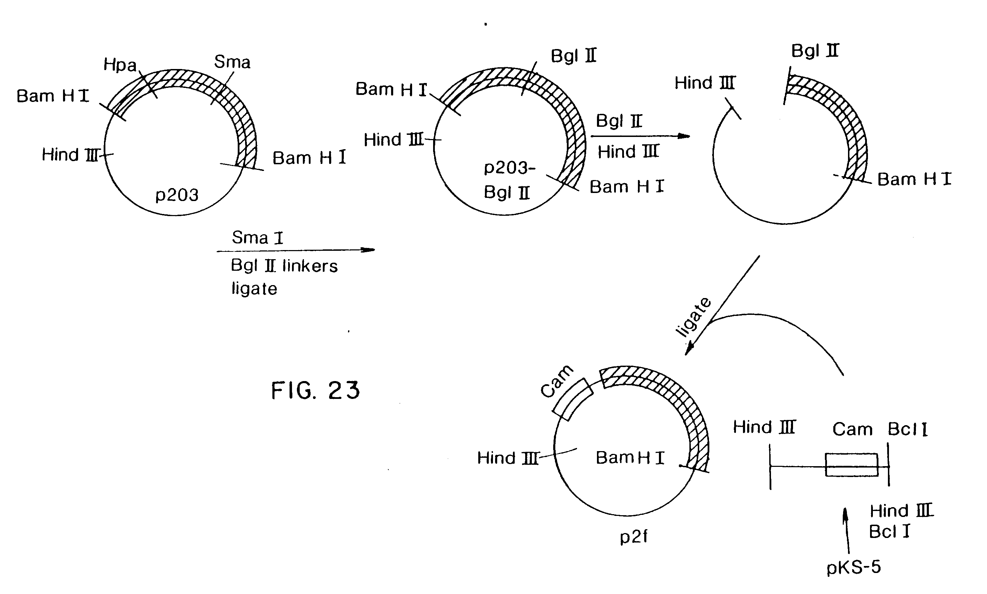

The Ti plasmid is mutated in this example by deleting the T-DNA between the *Hpa*l site in *tmr* to the *Sma*I site in *tml*. The Ti plasmids that can be modified include pTi15955, pTiB6, pTiA66 and others. This construction is diagramed in Fig. 23.

### 6.1 Isolation of the cam gene

pKS-5 (Fig. 19) is digested with *Hind*III and *Bcl*I. The smallest fragment is isolated after separation on an agarose gel, as taught in Example 5.

### 6.2 Construction of a pBR322 clone of T-DNA with a deletion

The right hand arm of the T-DNA deletion is constructed by insertion of a *Bgl*II site into the *Sma*l site of p203 (see Fig. 23). p203 is digested by *Sma*I*,* ligated with *Bgl*II linkers, digested with *Bgl*II, religated, and transformed into K802. In an alternative construction, *Bam*HI linkers are substituted for *Bgl*II linkers and the appropriate *Bam*HI partial digest products are isolated). The resultant plasmid is labeled p203-*Bgl*II, and is digested with *Bgl*II and *Hin*dIII. The large *Bgl*II*lHin*dIII vector containing fragment is ligated with the chloramphenicol resistance fragment whose isolation was described in Example 6.1. Chloramphenicol resistance is selected for after transformation into K802. The resultant plasmid is labeled p2f (Fig. 23).

### 6.3 Construction of left-hand arm of T-DNA deletion clone

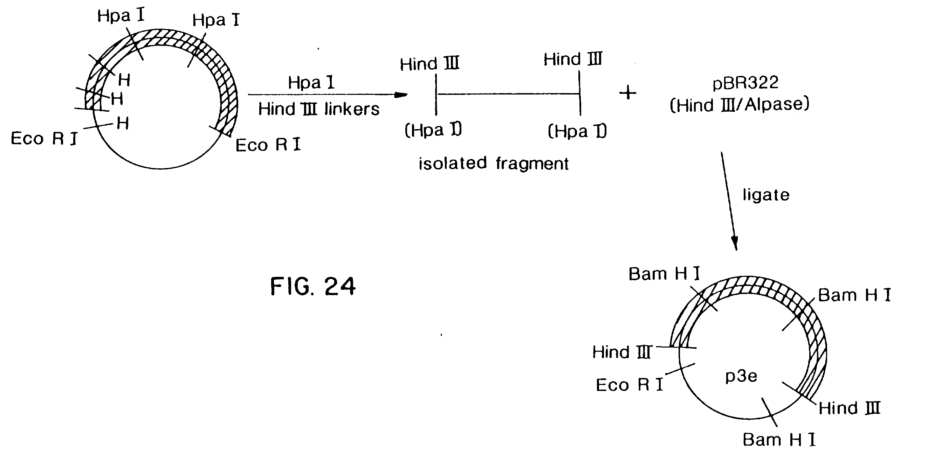

*Hin*dIII sites are inserted into the *Hpa*I site of p202 by digestion with *Hpa*I and ligation with *Hin*dIII linkers. After unmasking of the *Hin*dIII sticky ends by digestion with that restriction enzyme, the 2kbp *Hpa*I fragment which now bears *Hin*dIII ends is isolated. *Hin*dIII digested *Hin*dIII-ended *Hpa*I fragment and transformed into K802. After a colony containing the desired construction is isolated, and characterized, the plasmid is labeled p3e (Fig. 24).

### 6.4 Construction of the T-DNA deletion clone

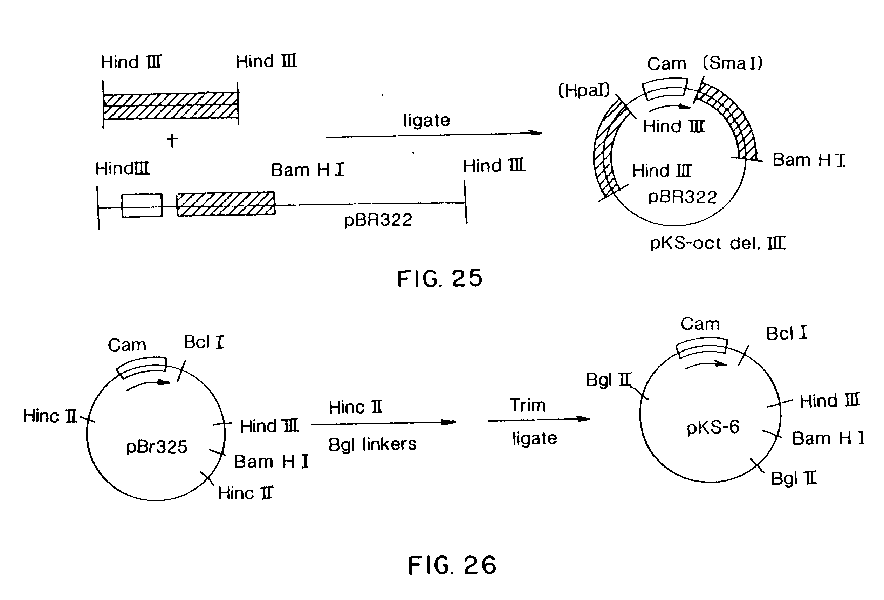

The left-hand arm of the clone is obtained by purifying a 2kbp fragment of a Hindlll digest of p3e by elution from an agarose gel after electrophoresis. p2f is cut by Hindlll, treated with alkaline phosphatase, mixed with the 2kbp fragment, ligated, transformed into K802, and selected for chloramphenicol resistance. Plasmids are isolated from individual colonies and characterized by restriction mapping. A plasmid having the two arms in the desired tandem orientation is chosen and labeled pKS-oct.dellll (Fig. 25).

pKS-Oct.delIII is moved into A. *tumefaciens by* mating, and homologous recombinants are selected by selection with chloramphenicol. Sunflower and tobacco roots and shoots are inoculated as described in other Examples and the tumors generated are tested for opines.

### Example 7

This example teaches a construction deleting *tmr* and *tml* that provides an alternative to that taught in Example 6.

### 7.1 Construction of a chloramphenicol resistant fragment with a BglII site

pBR325 is digested with *Hinc*II*,* blunt-end ligated with *Bgl*II linkers, digested with *Bgl*II, and religated (Fig. 26). Chloramphenicol resistance is selected for after transformation of either K802 or GM33. The resultant plasmid, pKS-6 serves as a source of the *Bgl*II*lBcl*I fragment carrying the *cam* gene.

### 7.2 Construction of the tmr, tml deletion clone

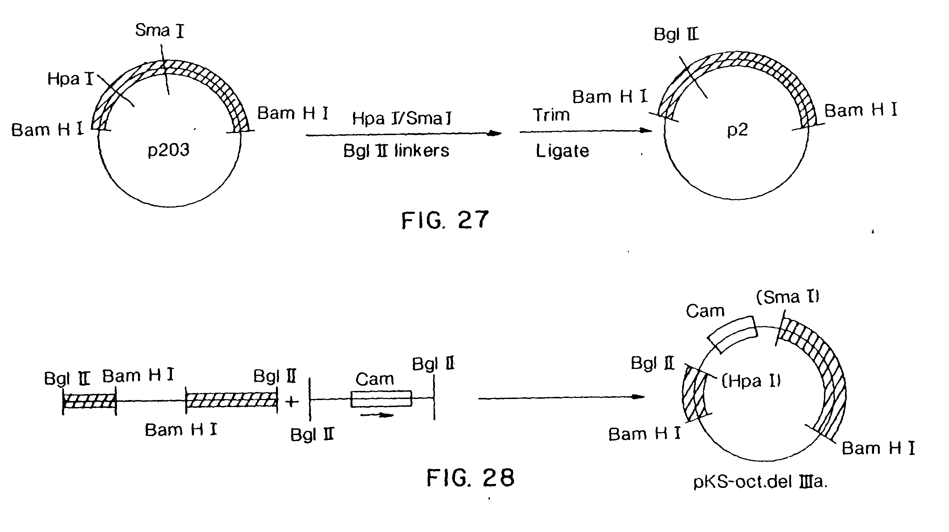

p203 is digested with *Hpa*I and *Sma*I*.* After blunt end ligation with *Bgl*II linkers, is it digested with *Bgl*II to expose the *Bgl*II sticky ends, religated and transformed into K802. The desired construction is identified and labeled p2 (Fig. 27).

### 7.3 Construction of the T-DNA deletion clone (pKS-oct.deIIIIa)

The *Bgl*II fragment carrying the *cam* gene is isolated from pKS-6 and ligated into *Bgl*II-cut p2. Chloramphenicol resistance is selected for after transformation of K802. The resultant plasmid is labeled pKS-oct.delllla (Fig. 28), and is tested as described in Example 6.4.

### Example 8

The purpose of this construction is to provide an example of the mutation of the *tmr* locus only at the *Hpa* site by insertion of the chloramphenicol resistance gene. This gene is isolated as the *Bgl*IIl*Bcl*I fragment from pKS-6, and is ligated into the *Hpal* site of p203 after that site is changed to a *Bgl*II site.

### 8.1 Conversion of the Hpa site to a BglII site

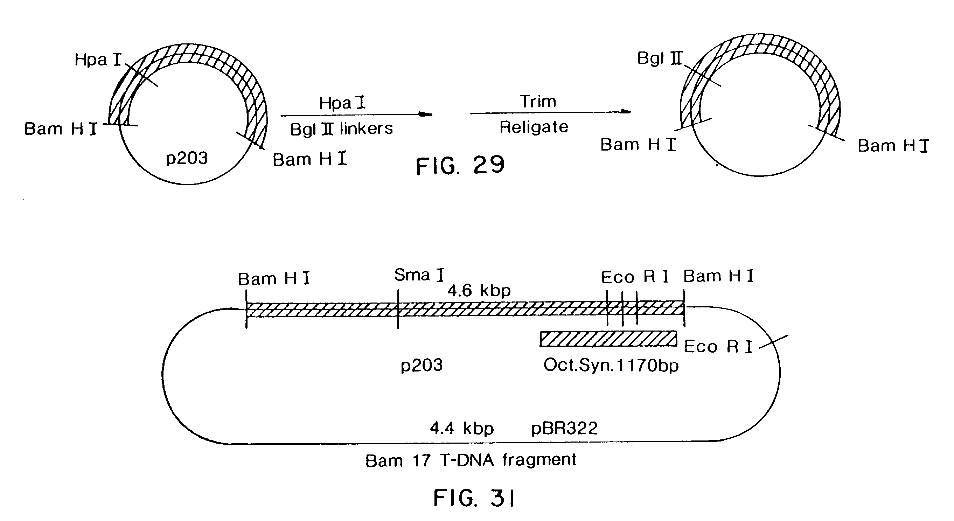

p203 is digested with *Hpa,* ligated to *Bgl*II linkers, trimmed with *Bgl*II and religated. After transformation of K802, colonies are selected and screened by restriction mapping for insertion of *Bgl*II sites (Fig. 29).

### 8.2 Isolation of the cam gene

pKS-6 is digested with *Bgl*II and *Bcl*I. The smallest fragment is isolated by agarose gel electrophoresis.

### 8.3 Construction of the mutated T-DNA clone

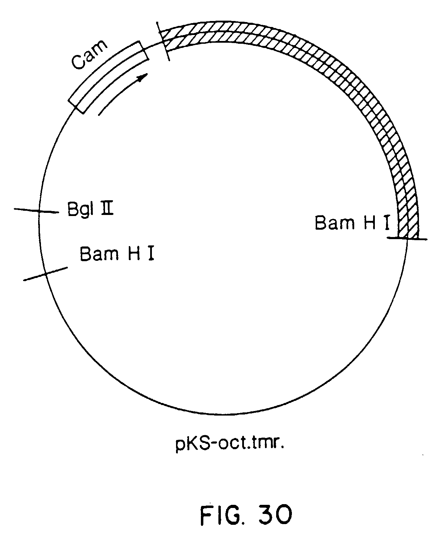

The modified p203 from Example 8.1 is digested with *Bgl*II, ligated with the purified *cam* gene from Example 8.2, and transformed into K802. Chloramphenicol resistance is selected for, and after isolation from the resistant transformants and characterization by restriction enzyme mapping, the plasmid is labeled pKS-oct.tmr (Fig. 30).

### Example 9

Regeneration in this example involves carrot tumors incited by an Ri-based TIP plasmid and is effected essentially as described by M.-D. Chilton *et al.* (1982) Nature *295*:432-434.

### 9.1 Infection with hairy root

Carrot disks are inoculated with about 10⁹ bacteria in 0.1 ml of water. One to 1.5 cm segments of the ends of the roots obtained are cut off, placed on solid 11-1.5% agar) Monier medium lacking hormones (D. A. Tepfer and J. C. Tempe (1981) C. R. Hebd. Seanc. Acad. Sci., Paris 295:153-156), and grown at 25°C to 27°C in the dark. Cultures uncontaminated by bacteria are transferred every 2 to 3 weeks and are subcultured in Monier medium lacking hormones and agar.

### 9.2 Regeneration of roots to plants

The cultured root tissue described in Example 9.1 is placed on solidified (0.8% agar) Monier medium supplemented with 0.36 µM 2,4-D and 0.72 µM kinetin. After 4 weeks, the resulting callus tissue is placed in liquid Monier medium lacking hormones. During incubation at 22 to 25°C on a shaker (150 rpm) for one month, the callus disassociates into a suspension culture from which embryos differentiate, which, when placed in Petri dishes containing Monier medium lacking hormone, develop into plantlets. These plantlets are grown in culture, and after "hardening" by exposure to atmospheres of progressively decreasing humidity, are transferred to soil in either a greenhouse or field plot.

### 9.3 Use of non-hairy root vectors

Ti-based vectors which do not have functional *tmr* genes are used instead of the Ri-based vectors as described in Examples 9.1 and 9.2. Construction of suitable deletions is described in Example 6, 7, and 8.

### Example 10

Regeneration in this example involves tobacco tumors incited by a Ti-based TIP plasmid and is effected essentially as described by K. A. Barton *et al.* (1983) Cell *32*:1033-1043.

### 10.1 Infection with crown gall

Tobacco tissue is transformed using an approach utilizing inverted stem segments first described by A. C. Braun (1956) Canc. Res. 16:53-56. Stems are surface sterilized with a solution that was 7% commercial Chlorox and 80% ethanol, rinsed with sterile distilled water, cut into 1 cm segments, placed basal end up in Petri dishes containing agar-solidified MS medium (T. Murashige and F. Skoog (1962) Physiol. Plant. *15*:473-497) lacking hormones. Inoculation is effected by puncturing the cut basal surface of the stem with a syringe needle and injecting bacteria. Stems are cultured at 25°C with 16 hours of light per day. The calli which develop are removed from the upper surface of the stem segments, are placed on solidified MS medium containing 0.2 mg/ml carbenicillin and lacking hormones, are transferred to fresh MS-carbenicillin medium three times at intervals of about a month, and are tested to ascertain whether the cultures had been ridden of bacteria. The axenic tissues are maintained on solidified MS media lacking supplements under the culture conditions (25°C; 16 hr.:8 hr. light:dark) described above.

### 10.2 Culture of transformed tissue

Clones are obtained from the transformed axenic tissues as described by A. Binns and F. Meins (1979) Planta *145*:365-369. Calli are converted into suspensions of cells by culturing in liquid MS having 0.02 mg/l naphthalene acetic acid (NAA) at 25°C for 2 or 3 days while being shaken at 135 rpm, and filtering in turn through 543 and 213 µm stainless steel meshes. The passed filtrate is concentrated, plated in 5 ml of MS medium containing 0.5% melted agar, 2.0 mg/l NAA, 0.3 mg/l kinetin and 0.4 g/l Difco yeast extract at a density of about 8×10³ cells/ml. Colonies reaching a diameter of about 1 mm are picked by scalpel point, placed onto and grown on solidified MS medium having 2.0 mg/l NAA and 0.3 mg/l kinetin. The resulting calli are split into pieces and tested for transformed phenotypes.

### 10.3 Regeneration of plants

Transformed clones are placed onto solidified MS medium having 0.3 mg/l kinetin, and cultured as described in Example 10.1. The shoots which form are rooted by putting them on a solid (1.0% agar) medium containing 1/10 strength MS medium salts, 0.4 mg/l thiamine, lacking sucrose and hormones, and having a pH of 7.0. Rooted plantlets are grown in culture, hardened as described in Example 9.2, and are transferred to soil in either a greenhouse or field plot.

### 10.4 Vectors used

The methods described in Examples 10.1, 10.2 and 10.3 are suitable Ti-based vectors lacking functional *tmr* genes. Construction of suitable deletions is described in Examples 6, 7, and 8. These methods are also effective when used with Ri-based vectors. The method described in Example 10.1 for infection of inverted stem segments is often useful for the establishment of TIP transformed plant cell lines.

### Example 11

Phaseolin is the most abundant storage protein (approximately 50% of the total seed protein) of *Phaseolis vulgaris.* Transfer of the functional phaseolin gene to alfalfa plants and translation of the phaseolin m-RNA into stored phaseolin is of significant economic value since it introduces storage protein synthesis into leaf material to be used as fodder. Alfalfa is a valuable plant for the transfer and expression of the phaseolin gene because of its acceptance as cattle fodder, its rapid growth, its ability to fix nitrogen through Rhizobial symbiosis, its susceptibility to crown gall infection and the ability to regenerate alfalfa plants from single cells or protoplasts. This example teaches the introduction of an expressible phaseolin gene into intact alfalfa plants.

### 11.1 Construction of shuttle vector

Alfalfa plants are regenerated from crown gall tissue containing genetically engineered *Agrobacterium* plasmids as described hereafter. In the first step we construct a "shuttle vector" containing a *tmr*⁻ and a *tms*^{*-*} T-DNA mutant linked to a functional phaseolin gene. This construction is, in turn, linked to a nopaline synthetase promoter which has a functional neomycin phosphotransferase (NPTII) structural gene (kanamycin resistance) downstream (reported by M.-D. Chilton, *et al.* (18 January 1983) 15th Miami Winter Symposium; see also J. L. Marx (1983) Science 219:830 and R. Horsch *et al.* (18 January 1983) 15th Miami Winter Symposium). This type of construction is illustrated in Example 1.

### 11.2 Transfer to Agrobacterium and plant cells

The "shuttle vector" is then transformed by conventional techniques (Example 14) into a strain of *Agrobacterium* containing a Ti plasmid such as pTi15955. Bacteria containing recombinant plasmids are selected and co-cultivated with alfalfa protoplasts which are regenerated cell walls (Marton *et al.* (1979) Nature *277*:129-131; G. J. Wullems *et al.* (1981) Proc. Nat. Acad. Sci. USA *78*:4344-4348; and R. B. Horsch and R. T. Fraley (18 January 1983) 15th Miami Winter Symposium).

Cells are grown in culture and the resulting callus tissue is tested for the presence of the appropriate mRNA by Northern blotting (Example 12) and for the presence of the appropriate proteins by ELISA tests (see J. L. Marx (1983) Science 219:830; R. B. Horsch and R. T. Fraley (18 January 1983) 15th Miami Winter Symposium).

### 11.3 Plant regeneration

Alfalfa plants are then regenerated from callus tissue by methods similar to those previously used by A. V. P. Dos Santos *et al.* (1980) Z. Pflanzenphysiol. *99*:261-270; T. J. McCoy and E. T. Bingham (1977) Plant Sci. Letters *10*:59-46; and K. A. Walker *et al.* (1979) Plant Sci. Letters *16*:23-30. These regenerated plants are then propagated by conventional plant breeding techniques forming the basis for new commercial varieties.

### Example 12

In all Examples, RNA was extracted, fractionated, and detected by the following procedures.

### 12.1 RNA extraction

This procedure was a modification of Silflow *et al*. (1981) Biochemistry *13*:2725-2731. Substitution of LiCI precipitation for CsC1 centrifugation was described by Murray *et al.* (1981) J. Mol. Evol. *17*:31-42. Use of 2 M LiCI plus 2 M urea to precipitate was taken from Rhodes (1975) J. Biol. Chem. *25*:8088-8097.

Tissue was homogenized using a polytron or ground glass homogenizer in 4-5 volumes of cold 50 mM Tris-HCI (pH 8.0) containing 4% p-amino salicylic acid, 1% tri-isopropyl napthalene sulfonic acid, 10 mM dithiothreitol (freshly made) and 10 mM Na-metabisulfite (freshly made). An octanol was used as needed to control foaming. An equal volume of Tris-saturated phenol containing 1% 8-hydroxyquinoline was added to the homogenate which was then shaken to emulsify and centrifuged at 20,000-30,000 g for 15 minutes at 4°C. The aqueous upper phase was extracted once with chloroform/octanol (24:1) and centrifuged as above. Concentrated LiCI-urea solution was then added to a final concentration of 2 M each and the mixture was left to stand at 20°C for several hours. The RNA precipitate was then centrifuged down and washed with 2 M LiCI to disperse the pellet. The precipitate was then washed with 70% ethanol-0.3 M Na-acetate and dissolved in sufficient sterile water to give a clear solution. One half volume of ethanol was added and the mixture put on ice for 1/2 hour, after which it was centrifuged to remove miscellaneous polysaccharides. The RNA precipitate was then recovered and redissolved in water or in sterile no salt poly(U) buffer.

### 12.2 Poly(U) sephadex chromotography

Two poly-U Sephadex (trademark: Pharmacia, Inc., Uppsala, Sweden) buffers were used; the first with no salt containing 20 mM Tris, 1 mM EDTA and 0.1% SDS, and the second with 0.1 M NaCl added to the first. In order to obtain a good match at A₂₆₀, a 2× stock buffer should be made and the salt added to a portion. After adjusting the final concentrations, the buffers are autoclaved.

Poly(U) Sephadex was obtained from Bethesda Research laboratories and 1 gm poly(U) Sephadex was used per 100 µg expected poly(A) RNA. The poly(U) Sephadex was hydrated in no salt poly(U) buffer and poured into a jacketed column. The temperature was raised to 60°C and the column was washed with no salt buffer until the baseline at 260 mm was flat. Finally the column was equilibrated with the salt containing poly(U) buffer at 40°C. The RNA at a concentration of less than 500 µg/ml was then heated in no salt buffer at 65°C for 5 minutes, after which it was cooled on ice and NaCI added to a concentration of 0.1 M. The RNA was then placed on the column which was run at no more than 1 ml/min until the optical density had fallen to a steady baseline. The column temperature was then raised to 60°C and the RNA was eluted with no salt poly(U) buffer. The RNA usually washed off in three column volumes. The eluted RNA was then concentrated with secondary butanol to a convenient volume after addition of NaCI to 10 mM, and precipitated with 2 volumes ethanol. The ethanol precipitate was dissolved in water and NH₄-acetate added to 0.1 M, followed by re-precipitation with ethanol. Finally the RNA was redissolved in sterile water and stored at -70°C.

### 12.3 Formaldehyde RNA gels and "northern" blots

The method used followed that of Thomas (1980) Proc. Nat. Acad. Sci. USA 77:5201, and Hoffman, *et al.* (1981) J. Biol. Chem. *256*:2597.

0.75-1.5% agarose gels containing 20 mM Na-phosphate (pH 6.8-7.0) were cast. If high molecular weight aggregate bands appeared, then the experiments were repeated with the addition of 6% or 2.2 M formaldehyde (use stock solution of 36%) to the gels. The formaldehyde caused visualization with ethidium bromide to be very difficult. The running buffer was 10 mM Na-phosphate (pH 6.8-7.0).

Prior to electrophoresis, the RNA was treated with a denaturing buffer having final concentrations of 6% formaldehyde, 50% formamide, 20 mM Na-phosphate buffer and 5 mM EDTA. The RNA was incubated in the buffer at 60°C for 10-20 minutes. The incubation was terminated by addition of stop buffer. For a 20 µl sample, 4 µl 50% glycerol, 10 mM EDTA, 5 mM Na-phosphate and bromphenol blue were added.

Submerged electrophoresis was used. The RNA was loaded before the gel was submerged, and run into the gel at 125 mA for 5 minutes. The gels were then submerged and the current reduced to 30 mA (overnight) or 50 mA (6-8 hours). The buffer was recirculated and the electrophoresis was done in a cold room.

### 12.4 "Northern" blots

If the gel was to be blotted to detect a specific RNA, it was not stained; a separate marker lane was used for staining. Staining was with 5 µg/ml ethidium bromide in 0.1 M Na-acetate and destaining was for several hours in 0.1 M Na-acetate. Treatment in water at 60-70°C for 5-10 minutes prior to staining helped visualization.

A gel to be blotted was soaked for 15 minutes in 10× standard saline citrate (SSC)-3% formaldehyde. If large RNA molecules were not eluting from the gel then a prior treatment in 50 mM NaOH for 10-30 minutes helped to nick the RNA. If base treatment was used, the gel was neutralized and soaked in SSC-formaldehyde before blotting. Transfer of the RNA to nitrocellulose was done by standard methods.

Prehybridization was done at 42°C for a minimum of 4 hours in 50% formamide, 10% dextran sulfate, 5× SSC, 5× Denhardt's, 100 µg/ml denatured carrier DNA, 20 µg/ml poly(A), 40 mM Na-phosphate (pH 6.8-7.0) and 0.2% SDS. Hybridization was done by addition of the probe to the same buffer with overnight incubation. The probe was not used at more than approximately 5×10⁵ cpm/ml.

After hybridization, the nitrocellulose was washed a number of times at 42°C with 2x SSC, 25 mM Na-phosphate, 5 mM EDTA and 2 mM Na-pyrophosphate followed by a final wash for 20 minutes at 64°C in 1 × SSC. Best results were obtained if the filter was not dried prior to autoradiography and the probe could be removed by extensive washing in 1 mM EDTA at 64°C.

### Example 13

"Western" blots, to detect antigens after SDS-polyacrylamide gel electrophoresis, were done essentially as described by R. P. Legocki and D. P. S. Verma (1981) Analyt. Biochem. *111*:385-392.

Micro-ELISA (Enzyme-Linked Immuno-Sorbant Assay) assays were done using Immulon-2 type plates with 96 wells by the following steps:

### 13.1 Binding antibody to plates

On Day 1, the wells were coated with 1:1000 dilution of antibody (rabbit antiphaseolin IgG) in coating buffer. 200 µl/well incubated at 37°C for 2-4 hours. The plates were covered with Saran Wrap. Then the plates were rinsed three times with phosphate buffered saline-Tween (PBS-Tween) allowing a 5-minute waiting period between each rinse step. Then 1% bovine serum albumin (BSA) was added to rinse and, after addition to the well, left to sit for 20 minutes before discarding. Rinsing was repeated five times more with PBS-Tween.

### 13.2 Tissue homogenization

The tissue was sliced up into small pieces and then homogenized with a polytron using 1 gm of tissue/ml phosphate buffered saline-Tween-2% polyvinyl pyrollidone-40 (PBS-Tween-2% PVP40). All samples were kept on ice before and after grinding and standard phaseolin curves were obtained. One standard curve was done in tissue homogenates and one standard curve was also done in buffer to check the recovery of phaseolin when ground in tissue. Following centrifugation of the homogenized samples, 100 µl of each sample was placed in a well and left overnight at 4°C. To avoid errors, duplicates of each sample were done. The plates were sealed during incubation.

### 13.3 Binding enzyme

After the overnight incubation, the antigen was discarded and the wells were washed five times with PBS-Tween allowing 5 minutes between each rinse.

A conjugate (rabbit anti-phaseolin IgG alkaline phosphatase linked) was then diluted 1:3000 in PBS-Tween-2% PVP containing 0.2% BSA and 150 µl was added to each well; followed by incubation for 3-6 hours at 37°C. After the incubation, the conjugate was discarded and the wells were rinsed five times with PBS-Tween, allowing five minutes between each rinse as before.

### 13.4 Assay

Immediately before running the assay, a 5 mg tablet of p-nitrophenyl phosphate (obtained from Sigma and stored frozen in the dark) was added per 10 ml substrate and vortexed until the tablet was dissolved. 200 µl of the room temperature solution was quickly added to each well. The reaction was measured at various times, e.g. t=0, 10, 20, 40, 60, 90 and 120 minutes, using a Dynatech Micro-ELISA reader. When p-nitrophenyl phosphate, which is colorless, was hydrolysed by alkaline phosphatase to inorganic phosphate and p-nitrophenol, the latter compound gave the solution a yellow color, which could be spectrophotometrically read at 410 nm. The lower limit of detection was less than 0.1 ng.

### Example 14

Triparental matings were generally accomplished as described below; other variations known to those skilled in the art are also acceptable. *E. coli*K802 (pRK290-based shuttle vector) was mated with *E. coli*(pRK2013) and an *A. tumefaciens* strain resistant to streptomycin. The pRK2013 transferred to the shuttle vector carrying strain and mobilized the shuttle vector for transfer to the *Agrobacterium.* Growth on a medium containing both streptomycin and the drug to which the shuttle vector is resistant, often either kanamycin or chloramphenicol, resulted in the selection of *Agrobacterium* cells containing shuttle vector sequences. A mating of these cells with *E. coli*(pPH1J1) resulted in the transfer of pPH1J1 to the *Agrobacterium* cells. pPH1J1 and pRK290-based shuttle vectors cannot coexist for long in the same cell. Growth on gentamycin, to which pPH1J1 carries a resistance gene, resulted in selection of cells having lost the pRK290 sequences. The only cells resistant to streptomycin, gentamycin, and either kanamycin or chloramphenicol are those which have Ti plasmids that have undergone double homologous recombination with the shuttle vector and now carry the desired construction.

The strains listed in Table 2 all resemble their parent strains in morphology. Similarly, the physiological characteristics of the listed strains are generally those of the parent strains, except where otherwise indicated in any of the foregoing description, Table 1 and the Figures.

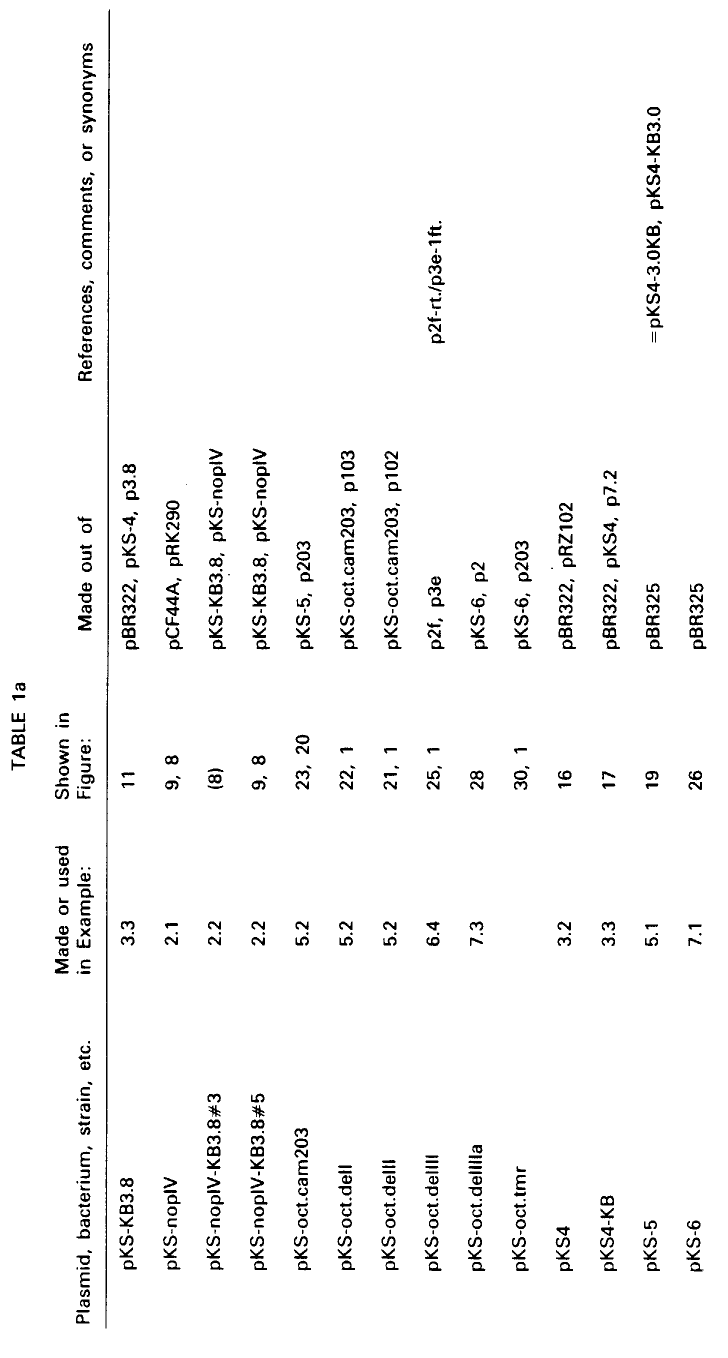

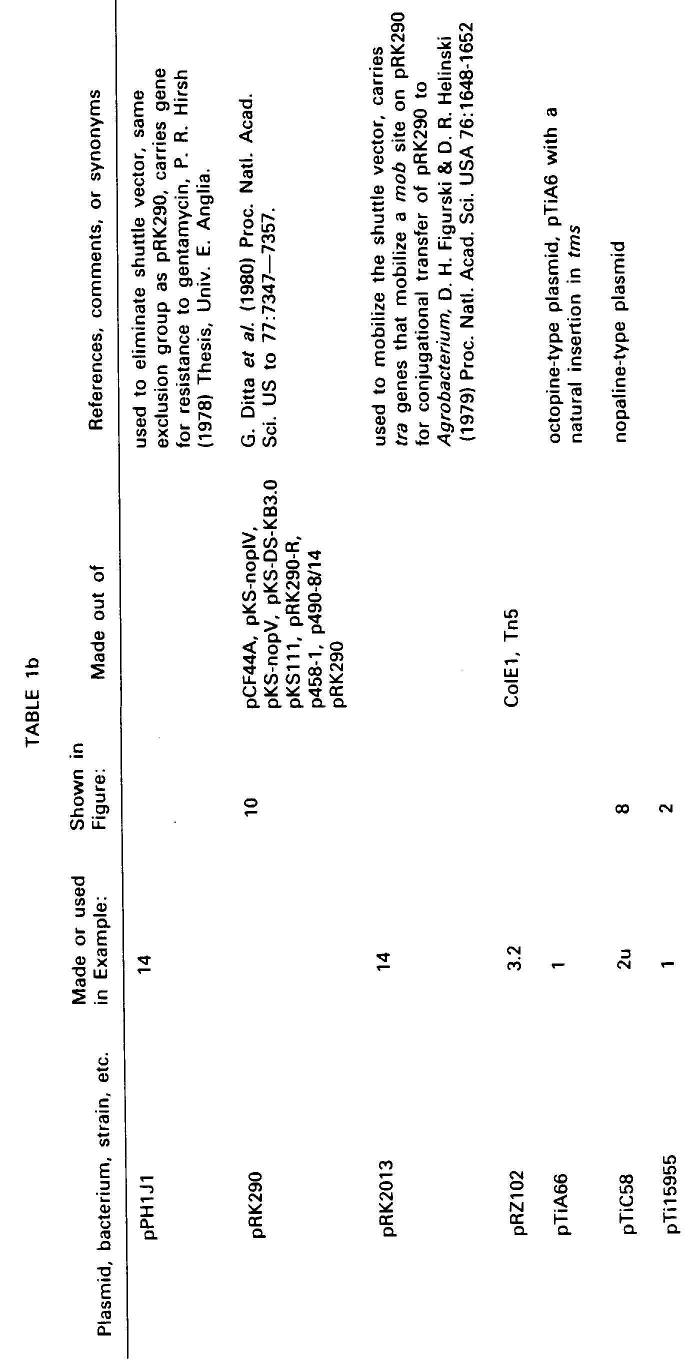

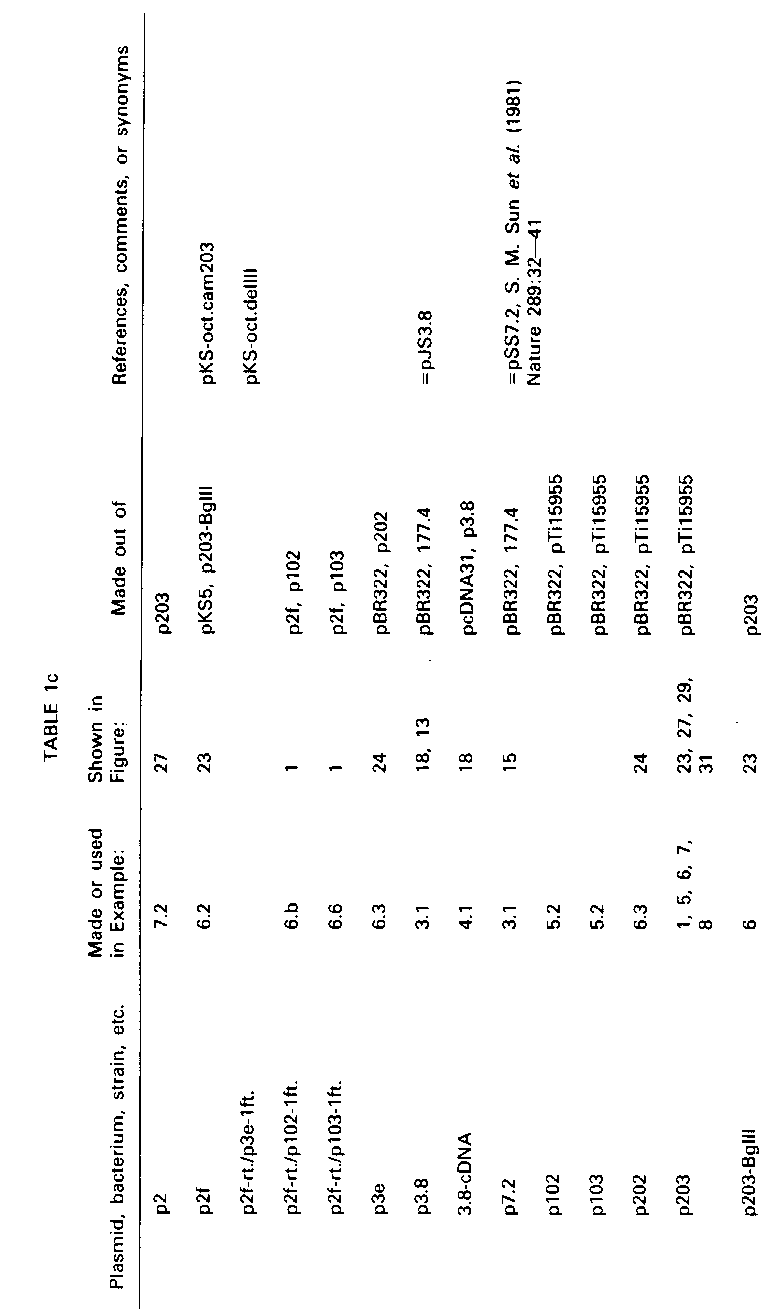

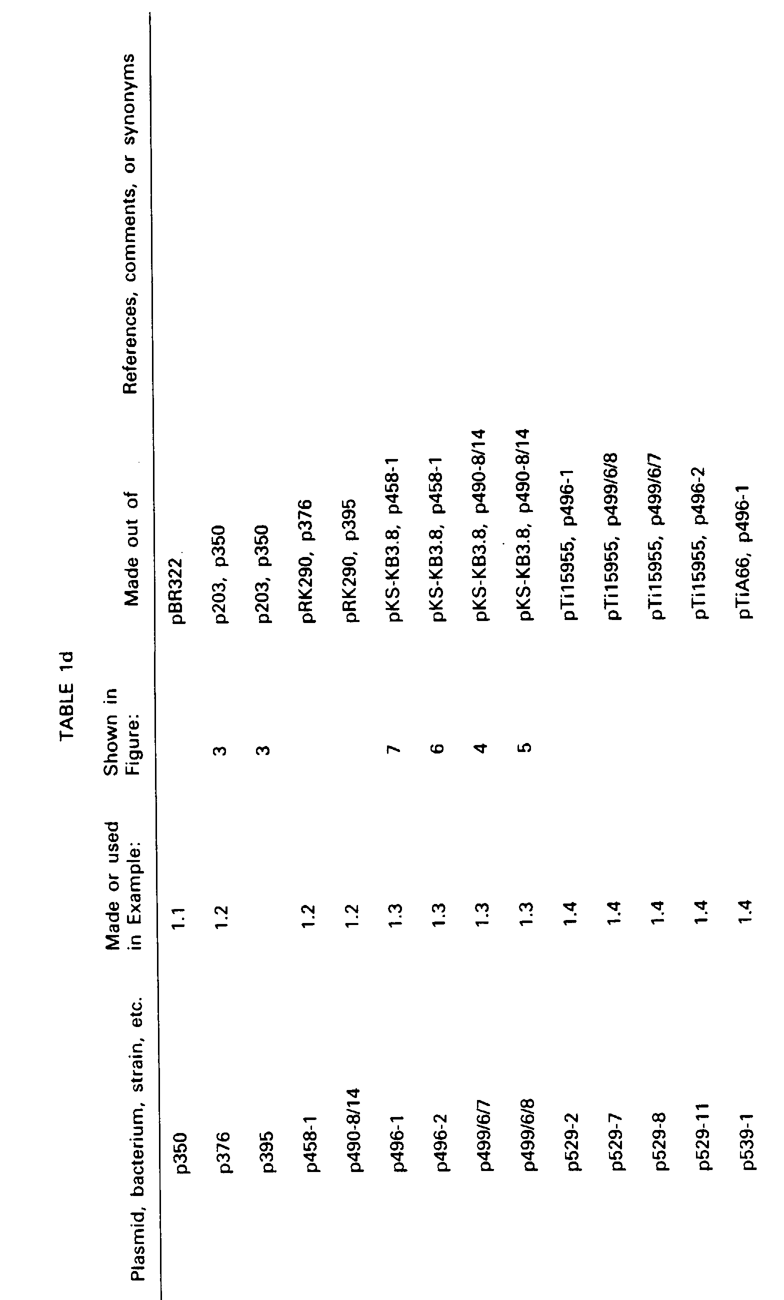

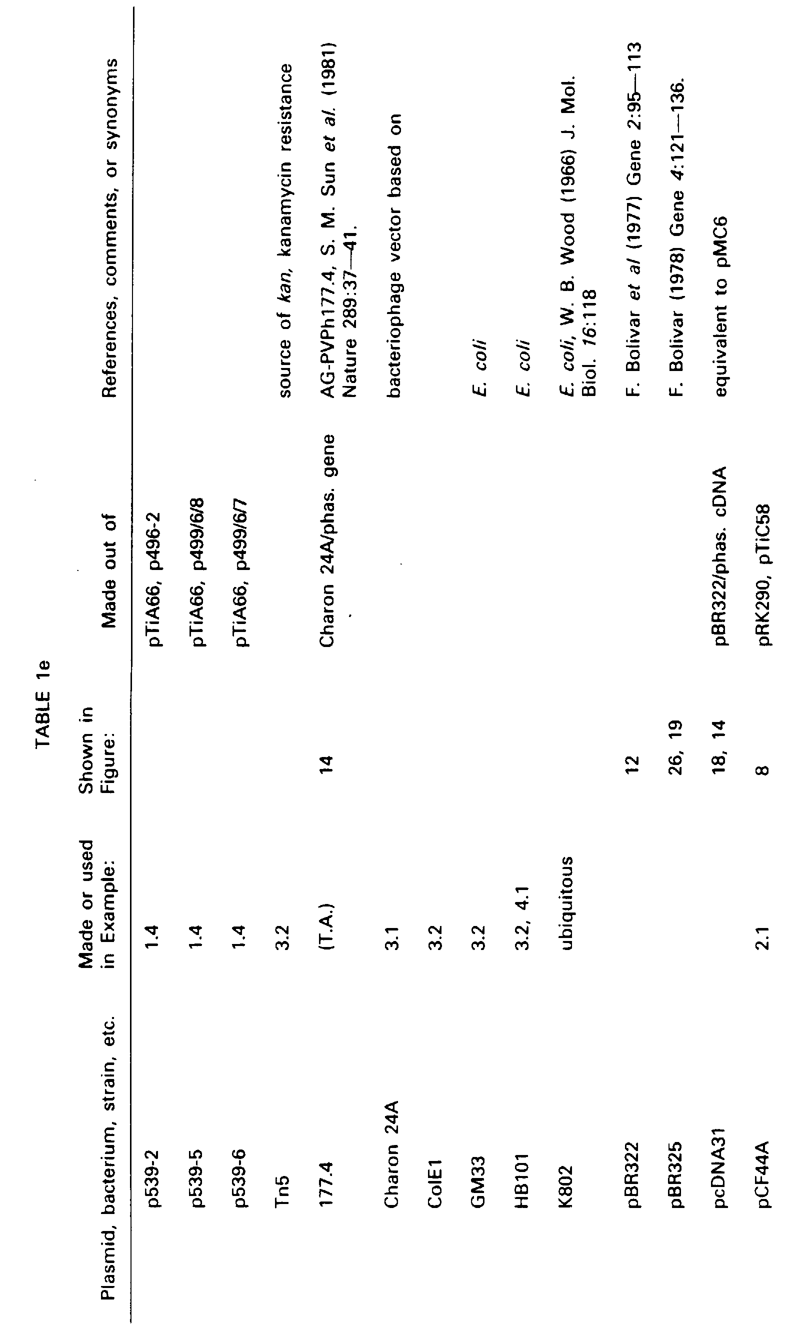

**TABLE 2**

| | |
|---|---|
| NRRL B-15372 | *A. tumefaciens*/p529-2 |
| NRRL B-15373 | *A. tumefaciens*/p529-8 |
| NRRL B-15374 | *A. tumefaciens*/p539-5 |
| NRRL B-15377 | *A. tumefaciens*/pC58-nop-KB#3 |
| NRRL B-15378 | *A. tumefaciens*/pC58-nop-KB#5 |
| NRRL B-15381 | *E. coli* K802/pKS-05-KB3.0 |
| NRRL B-15382 | *E. coli* K802/pKS-nopIV-KB3.8#3 |
| NRRL B-15383 | *E. coli* K802/pKS-nopIV-KB3.8#5 |
| NRRL B-15379 | *A. tumefaciens*/p529-11 |
| NRRL B-15384 | *E. coli* K802/p499/6/7 |
| NRRL B-15385 | *E. coli* K802/p499/6/8 |
| NRRL B-15386 | *E. coli* K802/p490-2 |
| NRRL B-15388 | *E. coli* K802/p490-1 |
| NRRL B.15389 | *E. coli* K802/p490-8/14 |
| NRRL B-15390 | *E. coli* K802/p458-8 |

## Claims

1. A method for genetically modifying a dicotyledonous plant cell, comprising the steps of:
(a) inserting a plant gene comprising a phaseolin promoter and a phaseolin structural gene into T-DNA, thereby forming a T-DNA/plant gene combination, the promoter being adjacent to the 5'-end of the structural gene and the structural gene being downstream from the promoter in the direction of transcription; and
(b) transferring the T-DNA/plant gene combination into a dicotyledonous plant cell, such that expression of the protein encoded by said structural gene is detectable in said plant cell.

2. A method according to claim 1, further comprising after execution of step (b) the step of:
(c) detecting expression of the protein encoded by the structural gene in a plant cell containing the T-DNA/plant gene combination.

3. A method according to claim 1, wherein the structural gene comprises one or more introns.

4. A method according to claim 1, wherein the T-DNA/plant gene combination is maintained and replicated prior to step (b) as part of a shuttle vector.

5. A method according to claim 1, wherein the plant gene is inserted into octopine-type T-DNA in tml or the "1.6" region.

6. A method according to claim 1, wherein the dicotyledonous plant is a member of the Compositeae or the Leguminoseae.

7. A plant cell produced according to the method of any of claims 1-6.

## Patentansprüche

1. Verfahren zur genetischen Modifizierung einer dicotyledonen Pflanzenzelle, welches folgende Schritte umfaßt:
(a) Einsetzen eines einen Phaseolin-Promotor und ein Phaseolin-Strukturgen umfassendes Pflanzengen in T-DNA, wodurch eine T-DNA/Pflanzengen-Kombination gebildet wird, wobei der Promotor dem 5'-Ende des Strukturgens benachbart ist und das Strukturgen in Transkriptionsrichtung stromabwärts des Promotors liegt; und
(b) Übertragen der T-DNA/Pflanzengen-Kombination in eine dicotyledone Pflanzenzelle, sodaß Expression des von diesem Strukturgen codierten Proteins in der genannten Pflanzenzelle feststellbar ist.

2. Verfahren nach Anspruch 1, welches nach Durchführung von Schritt (b) weiters folgenden Schritt umfaßt:
(c) Festellen der Expression des von dem Strukturgen codierten Proteins in einer die T-DNA/Pflanzengen-Kombination enthaltenden Pflanzenzelle.

3. Verfahren nach Anspruch 1, bei welchem das Strukturgen ein oder mehrere Introns umfaßt.

4. Verfahren nach Anspruch 1, bei welchem die T-DNA/Pflanzengen-Kombination vor Schritt (b) als Teil eines Shuttle-Vektors aufrechterhalten und repliziert wird.

5. Verfahren nach Anspruch 1, bei welchem das Pflanzengen in T-DNA vom Octopin-Typ in tml oder die "1.6"-Region eingesetzt wird.

6. Verfahren nach Anspruch 1, bei welchem die dikotyledone Pflanze ein Mitglied von Compositeae oder Leguminoseae ist.

7. Pflanzenzelle, hergestellt nach dem Verfahren nach einem der Ansprüche 1 bis 6.

## Revendications

1. Procédé pour modifier génétiquement une cellule de plante dicotylédone; comprenant les étapes suivantes:
(a) insertion d'un gène végétal comprenant un promoteur de phaséoline et un gène de structure de phaséoline dans un ADN-T, en formant ainsi une combinaison ADN-T/gène végétal, le promoteur étant adjacent à l'extrémité 5' du gène de structure et le gène de structure étant en aval du promoteur dans le sens de la transcription; et
(b) transfert de la combinaison ADN-T/gène végétal dans une cellule de plante dicotylédone, de sorte que l'expression de la protéine codée par ledit gène de structure soit décelable dans ladite cellule végétale.

2. Procédé selon la revendication 1, comprenant en outre, après l'exécution de l'étape (b), l'étape suivante:
(c) détection de l'expression de la protéine codée par le gène de structure dans une cellule végétale contenant la combinaison ADN-T/gène végétal.

3. Procédé selon la revendication 1, dans lequel le gène de structure comprend un ou plusieurs introns.

4. Procédé selon la revendication 1, dans lequel la combinaison ADN-T/gène végétal est maintenue et répliquée avant l'étape (b) dans un vecteur navette.

5. Procédé selon la revendication 1, dans lequel le gène végétal est inséré dans un ADN-T de type octopine dans la région tml ou "1,6".

6. Procédé selon la revendication 1, dans lequel la plante dicotylédone est un membre des Compositeae ou des Leguminoseae.

7. Cellule végétale produite selon le procédé de l'une quelconque des revendications 1-6.
